# EUROPEAN PATENT APPLICATION

(11) **EP 4 177 266 A1**
(43) Date of publication of application: **10.05.2023**
(21) Application number: 21206787.0
(22) Date of filing: 05.11.2021
(51) Int. Cl.: C07K 16/10, A61K 39/395, A61P 31/14, C12N 15/13

(54) **NEUTRALIZING ANTI-SARS-COV-2 HUMAN ANTIBODIES**

(71) Applicant: Katholieke Universiteit Leuven, 3000 Leuven (BE)
(72) Inventor: The designation of the inventor has not yet been filed

(57) **Abstract**

The invention relates to antibodies or antigen binding fragments thereof, specifically binding to the trimeric spike protein of Wuhan Covid-SARS-2 and specifically binding to the RBD domain of all of Wuhan, alpha, beta, gamma and delta variant of Covid-SARS-2 spike protein, characterized in that said antibody or antigen binding fragment thereof has an IC50 in a SARS-CoV-2 pseudovirus infection lower than 1 nm in a SARS-CoV-2 pseudovirus infection, and in that said antibody or antigen binding fragment thereof has an affinity for RBD domain Wuhan Covid-SARS-2 spike protein with a K_{D} of less than 500 pM, and in that said antibody or antigen binding fragment thereof has an affinity for trimeric spike protein of Wuhan Covid-SARS-2 with a K_{D} of less than 300 pM.

## Description

### FIELD OF THE INVENTION

The invention relates to antibodies which are particularly suitable in the treatment of beta and delta variant of COVID 19

### BACKGROUND OF THE INVENTION

The COVID-19 pandemic, caused by infection with severe acute respiratory syndrome coronavirus 2 (SARS-CoV-2), has resulted in an unprecedented global health and economic crisis and has already caused more than 4,8 million deaths worldwide¹. The spike protein of SARS-CoV-2 consists of an S1 subunit that recognizes host cell receptors and an S2 subunit that promotes membrane fusion of virus and host cells. Within the S1 subunit, the receptor binding domain (RBD) is responsible for interaction with receptor angiotensin-converting enzyme 2 (ACE2) on host cells to mediate viral entry. Consequently, SARS-CoV-2 spike protein is the major target of neutralizing antibodies (Ab)^{2,3.}

Antibodies can be elicited by natural infection and vaccination, or can be administered as recombinant monoclonal antibodies (mAbs) in a passive immunization strategy. Although vaccines are essential tools to fight this pandemic, therapeutic mAbs play a crucial role as well, especially for (immune-compromised) individuals who may not generate a robust response to their vaccine, cannot be vaccinated, are at high risk for severe illness or are still awaiting their vaccine⁴.

Until now, four mAb treatments have received emergency use authorization: three in the United States (REGN-COV2 (i.e. REGN10933 + REGN10987) from Regeneron, LY-CoV555 with/without LY-CoV016 from AbCellera/Eli Lilly and VIR-7831 from Vir Biotechnology/GlaxoSmithKline) and one in South Korea (CT-P59 from Celltrion)⁵. Unfortunately, SARS-CoV-2 variants beta, gamma and delta escape from some of the currently available therapeutic mAbs. REGN10933 (i.e. one of the antibodies from the REGN-COV2 antibody cocktail) showed reduced activity against SARS-CoV-2 beta and gamma, and activity of CT-P59 was reduced against beta, gamma and delta. LY-CoV555 completely lost its activity against beta, gamma and delta⁶⁻¹¹. This demonstrates that several of the commercially available therapeutic mAbs lose their activity against the current SARS-CoV-2 variants, and that there is a need for additional potent mAbs that recognize a broad range of different SARS-CoV-2 variants.

### SUMMARY OF THE INVENTION

In this work, we therefore sought to identify potent human mAbs reactive to all current SARS-CoV-2 variants both *in vitro* and *in vivo.* We were able to generate highly potent and broadly neutralizing mAbs that are capable of treating SARS-CoV-2 Wuhan, beta and delta infection in Golden Syrian hamsters.

Therapeutic monoclonal antibodies (mAbs) are an essential tool to combat COVID-19 morbidity and mortality, caused by severe acute respiratory syndrome coronavirus 2 (SARS-CoV-2). Four mAbs have already received emergency use authorization either in the United States or South Korea. Unfortunately, several SARS-CoV-2 variants escape some of these therapeutic mAbs. Using PBMCs from convalescent patients and B cell receptor cloning, we identified six mAbs, classified in four different epitope groups, that potently neutralize live SARS-CoV-2 Wuhan, alpha, beta, gamma and delta infection *in vitro.* In addition, antibodies 3E6 and 3B8 potently cure infection with SARS-CoV-2 Wuhan, beta and delta in an *in vivo* hamster model when administered therapeutically at 5 mg/kg. Interestingly, antibody 3B8 still reduced infectious viral titers after therapeutic administration at only 0.2 mg/kg, highlighting its superior potency. Combined with its reactivity against all tested SARS-CoV-2 variants, our data mark 3B8 as a promising candidate to help fight COVID-19.

The invention is further summarized in the following statements:
1. An antibody or antigen binding fragment thereof, specifically binding to the trimeric spike protein of Wuhan Covid-SARS-2 and specifically binding to the RBD domain of all of Wuhan, alpha, beta, gamma and delta variant of Covid-SARS-2 spike protein, characterized in that:
   said antibody or antigen binding fragment thereof has an IC50 in a SARS-CoV-2 pseudovirus infection lower than 1 nm, lower than 0,8 nm, lower than 0,6 nm, lower than 0,4 nm or lower than 0,2 nm in a SARS-CoV-2 pseudovirus infection, and in that said antibody or antigen binding fragment thereof has an affinity for RBD domain Wuhan Covid-SARS-2 spike protein with a KD of less than 500 pM, less than 400 pM,
   less than 300 pM, less than 200 pM, less than 100 pM or less than 50 pM, and in that said antibody or antigen binding fragment thereof has an affinity for trimeric spike protein of Wuhan Covid-SARS-2 with a KD of less than 300 pM, less than 200 pM, less than 100 pM or less than 50 pM.
2. The antibody or antigen binding fragment thereof according to statement 1, wherein
   said antibody or antigen binding fragment thereof has an IC50, lower than 0,4, and said antibody or antigen binding fragment thereof has an affinity for RBD domain Wuhan Covid-SARS-2 spike protein with a KD of less than 400 pM, and,
   said antibody or antigen binding fragment thereof has an affinity for trimeric spike protein of Wuhan Covid-SARS-2 with a KD of less than 300 pM.
3. The antibody or antigen binding fragment thereof according to statement 1 or 2, wherein,
   said antibody or antigen binding fragment thereof has an IC50, lower than 0,05 and said antibody or antigen binding fragment thereof has an affinity for RBD domain Wuhan Covid-SARS-2 spike protein with a KD of less than 250 pM, and
   said antibody or antigen binding fragment thereof has an affinity for trimeric spike protein of Wuhan Covid-SARS-2 with a KD of less than 100 pM.
4. The antibody or antigen binding fragment thereof according to any one of statements 1 to 3, which is an antibody or antibody fragment comprising:
   a variable heavy chain wherein the sequence of the CDR1, CDR2 and CDR3 region consist respectively of the sequence of GYTFTSHY [SEQ ID NO: 1], IDSSGGGA [SEQ ID NO: 2], and AKAHSTNWYGWFDP [SEQ ID NO: 3]
   and comprising
   a variable light chain wherein the sequence of the CDR1, CDR2 and CDR3 region consist respectively of the sequence SSNIGSNT [SEQ ID NO: 4], NNN and AAWDDGLNGSGWV [SEQ ID NO: 5]
5. The antibody or antigen binding fragment thereof according to statement 4, wherein the variable domain comprises a VH region that has outside the CDR regions at least 80%, at least 90%, at least 95%, at least 97 %, or at least 99 % sequence identity with the amino acid sequence of SEQ ID NO: 22 and a VL region that has outside the CDR regions at least 80%, at least 90%, at least 95%, at least 97 %, or at least 99 % sequence identity to the amino acid sequence of SEQ ID NO: 24.
6. The antibody or antigen binding fragment thereof according to statement 1 or 2, which is an antibody or antibody fragment comprising:
   - a variable heavy chain wherein the sequence of the CDR1, CDR2 and CDR3 region consist respectively of the sequence of GVIVSRNY [SEQ ID NO: 6], IYSGGST [SEQ ID NO: 7] and ARWGPNYYGSGSDHYNSYGMDV [SEQ ID NO: 8] and comprising
   a variable light chain wherein the sequence of the CDR1, CDR2 and CDR3 region consist respectively of the sequence QGVSSSS [SEQ ID NO: 9], GAS and QQSGSSPQYT[SEQ ID NO: 10].
7. The antibody or antigen binding fragment thereof according to statement 6, wherein the variable domain comprises a VH region that has outside the CDR regions at least 80%, at least 90%, at least 95%, at least 97 %, or at least 99 % sequence identity with the amino acid sequence of SEQ ID NO: 26 and a VL region that has outside the CDR regions at least 80%, at least 90%, at least 95%, at least 97 %, or at least 99 % sequence identity to the amino acid sequence of SEQ ID NO: 28.
8. The antibody or antigen binding fragment thereof according to statement 1, which is an antibody or antibody fragment comprising:
   - a variable heavy chain wherein the sequence of the CDR1, CDR2 and CDR3 region consist respectively of the sequence of GFTFDDYA[SEQ ID NO: 11], VSWNSGTI [SEQ ID NO: 12] and AKDVSVYRFREQSYGMDV [SEQ ID NO: 13]
      and comprising
   - a variable light chain wherein the sequence of the CDR1, CDR2 and CDR3 region consist respectively of the sequence SSDIGGYNF [SEQ ID NO: 14], EVT, and SSYTSSSLVV [SEQ ID NO: 15].
9. The antibody or antigen binding fragment thereof according to statement 8, wherein the variable domain comprises a VH region that has outside the CDR regions at least 80%, at least 90%, at least 95%, at least 97 %, or at least 99 % sequence identity with the amino acid sequence of SEQ ID NO: 30 and a VL region that has outside the CDR regions at least 80%, at least 90%, at least 95%, at least 97 %, or at least 99 % sequence identity to the amino acid sequence of SEQ ID NO: 32.
10. The antibody or antigen binding fragment thereof according to statement 1, which is an antibody or antibody fragment comprising:
   a variable heavy chain wherein the sequence of the CDR1, CDR2 and CDR3 region consist respectively of the sequence of GFSLDDYG [SEQ ID NO: 16], ISWNSGDR [SEQ ID NO: 17], and AKGSGAHYDIMTGARLDY [SEQ ID NO: 18] and comprising a variable light chain wherein the sequence of the CDR1, CDR2 and CDR3 region consist respectively of the sequence QSITNY [SEQ ID NO: 19], AAS, CDR 3: QQSYSTPWT [SEQ ID NO: 20].
11. The antibody or antigen binding fragment thereof according to statement 10, wherein the variable domain comprises a VH region that has outside the CDR regions at least 80%, at least 90%, at least 95%, at least 97 %, or at least 99 % sequence identity with the amino acid sequence of SEQ ID NO: 34 and a VL region that has outside the CDR regions at least 80%, at least 90%, at least 95%, at least 97 %, or at least 99 % sequence identity to the amino acid sequence of SEQ ID NO: 36.
12. The antibody or antigen binding fragment thereof according to statement 1, which is an antibody or antibody fragment comprising:
   a variable heavy chain wherein the sequence of the CDR1, CDR2 and CDR3 region consist respectively of the sequence of GYSFTSHW [SEQ ID. NO: 37], IFPGDSKT [SEQ ID. NO: 38], and ARPNRYYDDSGNYDGSYYFDY [SEQ ID. NO: 39],
   a variable light chain wherein the sequence of the CDR1, CDR2 and CDR3 region consist respectively of the sequence QSVNTN [SEQ ID. NO: 40], GAF, and QQYNNWPPAWT [SEQ ID. NO: 41].
13. The antibody or antigen binding fragment thereof according to statement 12, wherein the variable domain comprises a VH region that has outside the CDR regions at least 80%, at least 90%, at least 95%, at least 97 %, or at least 99 % sequence identity with the amino acid sequence of SEQ ID NO: 58 and a VL region that has outside the CDR regions at least 80%, at least 90%, at least 95%, at least 97 %, or at least 99 % sequence identity to the amino acid sequence of SEQ ID NO: 60.
14. The antibody or antigen binding fragment thereof according to statement 1, which is an antibody or antibody fragment comprising:
   a variable heavy chain wherein the sequence of the CDR1, CDR2 and CDR3 region consist respectively of the sequence of GYTFTTYY [SEQ ID. NO: 42], INSSGGST[SEQ ID. NO: 43], and ATSGWYRGAFDY [SEQ ID. NO: 44]
   a variable light chain wherein the sequence of the CDR1, CDR2 and CDR3 region consist respectively of the sequence SGSIDNNY [SEQ ID. NO: 45], EDN, and QSIDSNSWV [SEQ ID. NO: 46].
15. The antibody or antigen binding fragment thereof according to statement 14, wherein the variable domain comprises a VH region that has outside the CDR regions at least 80%, at least 90%, at least 95%, at least 97 %, or at least 99 % sequence identity with the amino acid sequence of SEQ ID NO: 62 and a VL region that has outside the CDR regions at least 80%, at least 90%, at least 95%, at least 97 %, or at least 99 % sequence identity to the amino acid sequence of SEQ ID NO: 64.
16. The antibody or antigen binding fragment thereof according to statement 1, which is an antibody or antibody fragment comprising:
   a variable heavy chain wherein the sequence of the CDR1, CDR2 and CDR3 region consist respectively of the sequence of GGSLSVYY [SEQ ID. NO: 47], INHSGST [SEQ ID. NO: 48], and ARTSLHTWNPFDY [SEQ ID. NO: 49], and
   a variable light chain wherein the sequence of the CDR1, CDR2 and CDR3 region consist respectively of the sequence QGISSY [SEQ ID. NO: 50], AAS, and QQLNNYPLT [SEQ ID. NO: 51].
17. The antibody or antigen binding fragment thereof according to statement 16, wherein the variable domain comprises a VH region that has outside the CDR regions at least 80%, at least 90%, at least 95%, at least 97 %, or at least 99 % sequence identity with the amino acid sequence of SEQ ID NO: 66 and a VL region that has outside the CDR regions at least 80%, at least 90%, at least 95%, at least 97 %, or at least 99 % sequence identity to the amino acid sequence of SEQ ID NO: 68.
18. The antibody or antigen binding fragment thereof according to statement 1, which is an antibody or antibody fragment comprising:
   a variable heavy chain wherein the sequence of the CDR1, CDR2 and CDR3 region consist respectively of the sequence of GGTFSNYA [SEQ ID. NO: 52], IIAIFGTP [SEQ ID. NO: 53], and ARRSTILERLPRHYYGMDV [SEQ ID. NO: 54], and
   a variable light chain wherein the sequence of the CDR1, CDR2 and CDR3 region consist respectively of the sequence QSLLHSNGNIY [SEQ ID. NO: 55], LGS and MQTLQTLRAT [SEQ ID. NO: 56].
19. The antibody or antigen binding fragment thereof according to statement 18, wherein the variable domain comprises a VH region that has outside the CDR regions at least 80%, at least 90%, at least 95%, at least 97 %, or at least 99 % sequence identity with the amino acid sequence of SEQ ID NO: 70 and a VL region that has outside the CDR regions at least 80%, at least 90%, at least 95%, at least 97 %, or at least 99 % sequence identity to the amino acid sequence of SEQ ID NO: 72.
20. The antibody or antigen binding fragment thereof according to any one of statements 1 to 19, wherein the antibody is a human antibody.
21. A polynucleotide encoding the antibody or antigen binding fragment thereof in accordance with any one of statements 1 to 20.
22. The antibody or antigen binding fragment thereof according to any one of statements 1 to 20, for use in the treatment and prevention of COVID 19 in an individual.
23. The antibody antigen binding fragment thereof according to any one of statements 22, for use in the treatment or prevention of COVID 19 in an individual, wherein only one antibody is administered to said individual.
24. The antibody antigen binding fragment thereof for use in the treatment or prevention of COVID 19 in an individual according to statement 22 or 23, wherein said antibody is administered intravenous or intramuscularly, in an amount from between 100 mg, 500 mg, or 1000 mg Ab per individual up to 2000, 4000, 6000 or 10000 mg per individual [all ranges with one of the mentioned lower and upper concentrations are herewith explicitly disclosed].
25. The antibody antigen binding fragment thereof for use in the treatment or prevention of COVID 19 in an individual according to any one of statements 22 to 24, wherein COVID 19 is the beta or delta variant of COVID 19.
26. The antibody antigen binding fragment thereof for use in the treatment or prevention of COVID 19 in an individual according to any one of statements 22 to 25, wherein the antibody or antibody fragment comprises:
   - a variable heavy chain wherein the sequence of the CDR1, CDR2 and CDR3 region consist respectively of the sequence of GYTFTSHY [SEQ ID NO: 1], IDSSGGGA [SEQ ID NO: 2], and AKAHSTNWYGWFDP [SEQ ID NO: 3]
   and comprising
   a variable light chain wherein the sequence of the CDR1, CDR2 and CDR3 region consist respectively of the sequence SSNIGSNT [SEQ ID NO: 4], NNN and AAWDDGLNGSGWV [SEQ ID NO: 5]
27. The antibody antigen binding fragment thereof for use in the treatment or prevention of COVID 19 in an individual according to any one of statements 22 to 25, wherein the variable domain comprises a VH region that has outside the CDR regions at least 80%, at least 90%, at least 95%, at least 97 %, or at least 99 % sequence identity with the amino acid sequence of SEQ ID NO: 22 and a VL region that has outside the CDR regions at least 80%, at least 90%, at least 95%, at least 97 %, or at least 99 % sequence identity to the amino acid sequence of SEQ ID NO: 24.
28. The antibody antigen binding fragment thereof for use in the treatment or prevention of COVID 19 in an individual according to any one of statements 22 to 25, comprising
   a variable heavy chain wherein the sequence of the CDR1, CDR2 and CDR3 region consist respectively of the sequence of GVIVSRNY [SEQ ID NO: 6], IYSGGST [SEQ ID NO: 7] and ARWGPNYYGSGSDHYNSYGMDV [SEQ ID NO: 8] and comprising
   a variable light chain wherein the sequence of the CDR1, CDR2 and CDR3 region consist respectively of the sequence QGVSSSS [SEQ ID NO: 9], GAS and QQSGSSPQYT[SEQ ID NO: 10]
29. The antibody or antigen binding fragment thereof for use in the treatment or prevention of COVID 19 in an individual according to any one of statements 22 to 25, wherein the variable domain comprises a VH region that has outside the CDR regions at least 80%, at least 90%, at least 95%, at least 97 %, or at least 99 % sequence identity with the amino acid sequence of SEQ ID NO: 26 and a VL region that has outside the CDR regions at least 80%, at least 90%, at least 95%, at least 97 %, or at least 99 % sequence identity to the amino acid sequence of SEQ ID NO: 28.
30. The antibody or antigen binding fragment thereof for use in the treatment or prevention of COVID 19 in an individual according to any one of statements 22 to 25, which is an antibody or antibody fragment comprising:
   a variable heavy chain wherein the sequence of the CDR1, CDR2 and CDR3 region consist respectively of the sequence of GYSFTSHW [SEQ ID. NO: 37], IFPGDSKT [SEQ ID. NO: 38], and ARPNRYYDDSGNYDGSYYFDY [SEQ ID. NO: 39],
   a variable light chain wherein the sequence of the CDR1, CDR2 and CDR3 region consist respectively of the sequence QSVNTN [SEQ ID. NO: 40], GAF, and QQYNNWPPAWT [SEQ ID. NO: 41].
31. The antibody or antigen binding fragment thereof for use in the treatment or prevention of COVID 19 in an individual according to any one of statements 22 to 25, wherein the variable domain comprises a VH region that has outside the CDR regions at least 80%, at least 90%, at least 95%, at least 97 %, or at least 99 % sequence identity with the amino acid sequence of SEQ ID NO: 58 and a VL region that has outside the CDR regions at least 80%, at least 90%, at least 95%, at least 97 %, or at least 99 % sequence identity to the amino acid sequence of SEQ ID NO: 60.
32. The antibody or antigen binding fragment thereof for use in the treatment or prevention of COVID 19 in an individual according to any one of statements 22 to 25, which is an antibody or antibody fragment comprising:
   a variable heavy chain wherein the sequence of the CDR1, CDR2 and CDR3 region consist respectively of the sequence of GYTFTTYY [SEQ ID. NO: 42], INSSGGST[SEQ ID. NO: 43], and ATSGWYRGAFDY [SEQ ID. NO: 44]
   a variable light chain wherein the sequence of the CDR1, CDR2 and CDR3 region consist respectively of the sequence SGSIDNNY [SEQ ID. NO: 45], EDN, and QSIDSNSWV [SEQ ID. NO: 46].
33. The antibody or antigen binding fragment thereof for use in the treatment or prevention of COVID 19 in an individual according to any one of statements 22 to 25, wherein the variable domain comprises a VH region that has outside the CDR regions at least 80%, at least 90%, at least 95%, at least 97 %, or at least 99 % sequence identity with the amino acid sequence of SEQ ID NO: 62 and a VL region that has outside the CDR regions at least 80%, at least 90%, at least 95%, at least 97 %, or at least 99 % sequence identity to the amino acid sequence of SEQ ID NO: 64.
34. The antibody or antigen binding fragment thereof for use in the treatment or prevention of COVID 19 in an individual according to any one of statements 22 to 25, which is an antibody or antibody fragment comprising:
   a variable heavy chain wherein the sequence of the CDR1, CDR2 and CDR3 region consist respectively of the sequence of GGSLSVYY [SEQ ID. NO: 47], INHSGST [SEQ ID. NO: 48], and ARTSLHTWNPFDY [SEQ ID. NO: 49], and
   a variable light chain wherein the sequence of the CDR1, CDR2 and CDR3 region consist respectively of the sequence QGISSY [SEQ ID. NO: 50], AAS, and QQLNNYPLT [SEQ ID. NO: 51].
35. The antibody or antigen binding fragment thereof for use in the treatment or prevention of COVID 19 in an individual according to any one of statements 22 to 25, wherein the variable domain comprises a VH region that has outside the CDR regions at least 80%, at least 90%, at least 95%, at least 97 %, or at least 99 % sequence identity with the amino acid sequence of SEQ ID NO: 66 and a VL region that has outside the CDR regions at least 80%, at least 90%, at least 95%, at least 97 %, or at least 99 % sequence identity to the amino acid sequence of SEQ ID NO: 68.
36. The antibody or antigen binding fragment thereof for use in the treatment or prevention of COVID 19 in an individual according to any one of statements 22 to 25, which is an antibody or antibody fragment comprising:
   a variable heavy chain wherein the sequence of the CDR1, CDR2 and CDR3 region consist respectively of the sequence of GGTFSNYA [SEQ ID. NO: 52], IIAIFGTP [SEQ ID. NO: 53], and ARRSTILERLPRHYYGMDV [SEQ ID. NO: 54], and
   a variable light chain wherein the sequence of the CDR1, CDR2 and CDR3 region consist respectively of the sequence QSLLHSNGNIY [SEQ ID. NO: 55], LGS and MQTLQTLRAT [SEQ ID. NO: 56].
37. The antibody or antigen binding fragment thereof for use in the treatment or prevention of COVID 19 in an individual according to any one of statements 22 to 25, wherein the variable domain comprises a VH region that has outside the CDR regions at least 80%, at least 90%, at least 95%, at least 97 %, or at least 99 % sequence identity with the amino acid sequence of SEQ ID NO: 70 and a VL region that has outside the CDR regions at least 80%, at least 90%, at least 95%, at least 97 %, or at least 99 % sequence identity to the amino acid sequence of SEQ ID NO: 72.
38. A polynucleotide comprising a sequence encoding the antibody or antigen binding fragment thereof according to any one of statements 1 to 20, for use in the treatment and prevention of COVID 19. herein "a" polynucleotide can be a single polynucleotide strand comprising both the sequences encoding the light and heavy chain or fragments of these.
   Alternatively two polynucleotide strands (e.g. plasmids)are administered, wherein one polynucleotide expresses the light chain or fragments thereof, and a second polynucleotide expresses the heavy chain or fragments thereof.
39. The polynucleotide for use in the treatment or prevention of COVID 19 in an individual according to statement 38, wherein COVID 19 is the beta or delta variant of COVID 19.
40. The polynucleotide for use in the treatment or prevention of COVID 19 in an individual according to statements 38 or 39, comprising the sequence of SEQ ID NO: 21 and SEQ ID NO: 23.
41. The polynucleotide for use in the treatment or prevention of COVID 19 in an individual according to statements 38 or 39, comprising the sequence of SEQ ID NO: 25 and SEQ ID NO: 27.
42. The polynucleotide for use in the treatment or prevention of COVID 19 in an individual according to statements 38 or 39, comprising the sequence of SEQ ID NO: 29 and SEQ ID NO: 31.
43. The polynucleotide for use in the treatment or prevention of COVID 19 in an individual according to statements 38 or 39, comprising the sequence of SEQ ID NO: 33 and SEQ ID NO: 35.
44. The polynucleotide for use in the treatment or prevention of COVID 19 in an individual according to statements 38 or 39, comprising the sequence of SEQ ID NO: 57 and SEQ ID NO: 59.
45. The polynucleotide for use in the treatment or prevention of COVID 19 in an individual according to statements 38 or 39, comprising the sequence of SEQ ID NO: 61 and SEQ ID NO: 63.
46. The polynucleotide for use in the treatment or prevention of COVID 19 in an individual according to statements 38 or 39, comprising the sequence of SEQ ID NO: 65 and SEQ ID NO: 67.
47. The polynucleotide for use in the treatment or prevention of COVID 19 in an individual according to claims 38 or 39, comprising the sequence of SEQ ID NO: 69 and SEQ ID NO: 71.

### DETAILED DESCRIPTION

### Figure legends

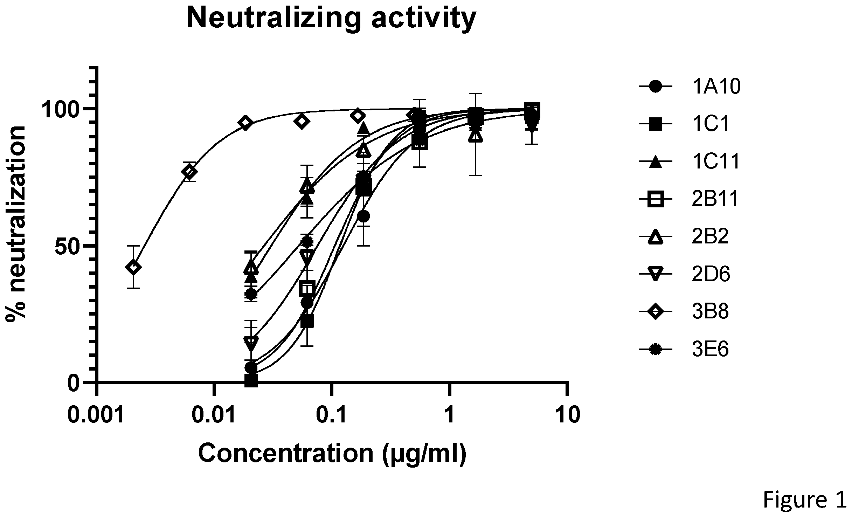

**Figure 1****: Neutralizing activity of top 8 most potent antibodies.** Percentage neutralizing activity, measured via a SARS-CoV-2 D614G VSV pseudotyped assay, is given at different concentrations for the top 8 antibodies. Symbols show mean and standard deviation of 3 replicates, graph is representative for 2 independent experiments.

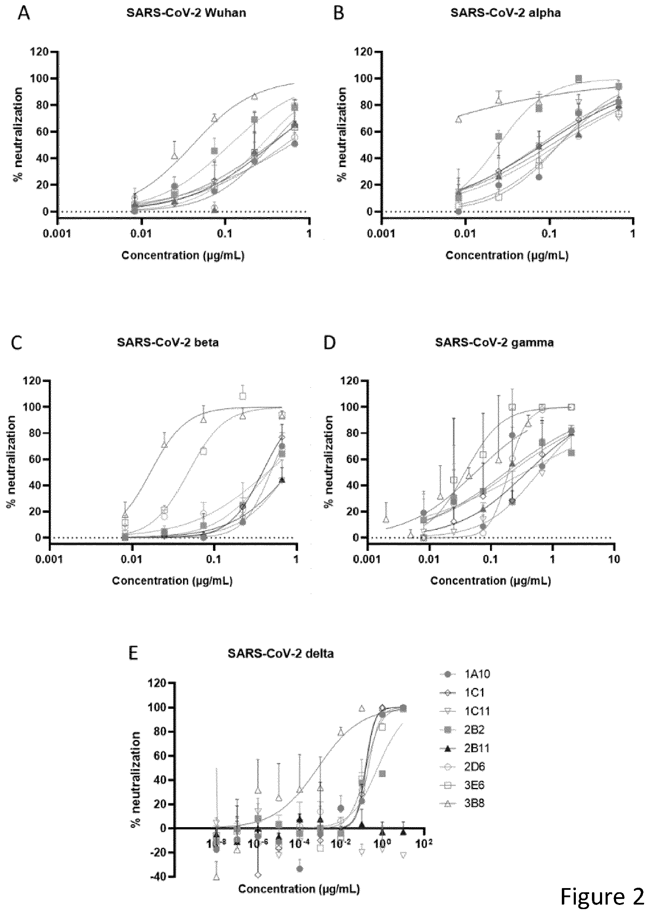

**Figure 2****: *in vitro* neutralization of infection with live SARS-CoV-2 variants.** Concentration-dependent neutralizing activity (% neutralization) was measured via CPENT assay using SARS-CoV-2 Wuhan, alpha, beta and gamma (A-D) or via PRNT assay using SARS-CoV-2 delta (E) for the 8 selected antibodies. Symbols show mean and standard deviation (only upper bar is shown for clarity) of 3 (A-D) or 2 (E) replicates.

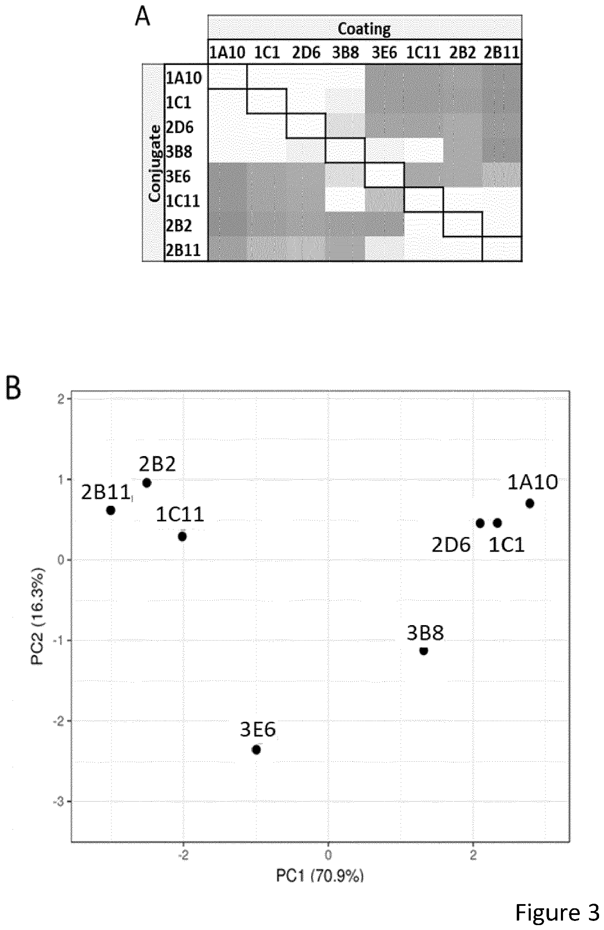

**Figure 3****: Epitope specificity of top 8 mAbs.** A) Visual representation of the cross-competition ELISA. All antibodies were pairwise tested against one another as coating or detection antibody. Signal strength is visualized by color intensity, with a white color indicating no binding of the detection antibody could occur, and dark red indicating strong binding of the detection antibody. B) Principal component analysis of the results obtained in the cross-competition ELISA to enable classification of the antibodies in different epitope groups. Data are representative for 2 independent experiments.

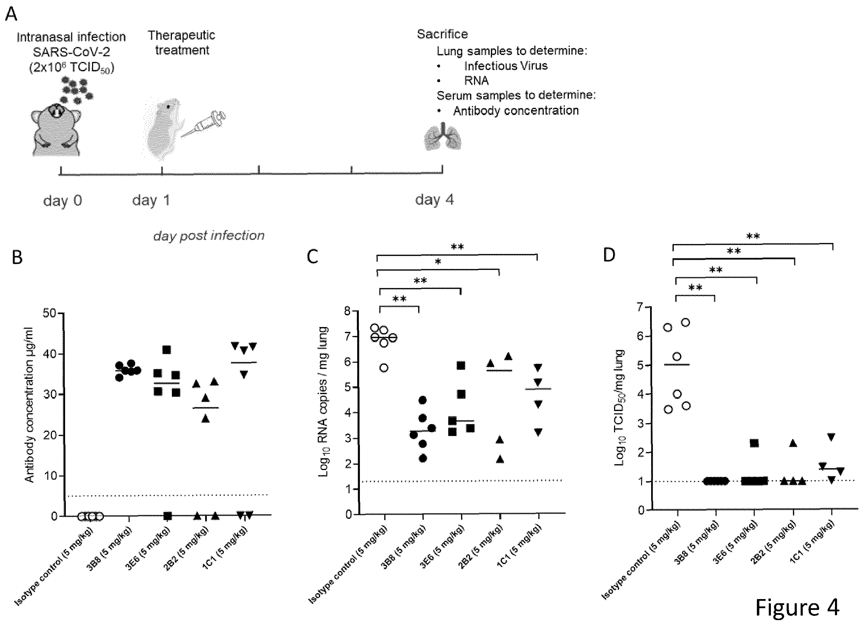

**Figure 4****: Treatment of SARS-CoV-2 Wuhan infection by antibodies 3B8, 3E6, 2B2 and 1C1 (5 mg/kg) in hamsters.** A) Study design. Hamsters were intranasally infected with SARS-CoV-2 on day 0 and received antibody treatment (5 mg/kg) 24h post infection. Animals were sacrificed at day 4 for analysis of lung and blood samples. B) Concentration (µg/ml) of the administered antibodies in serum at day 4 post infection. Animals without detectable antibody serum levels were excluded from the graphs in panel C and D. C) Viral RNA levels, expressed as log₁₀ RNA copies per mg of lung tissue, on day 4 post infection. D) Infectious viral loads, expressed as log₁₀ TCID₅₀ per mg lung tissue, at day 4 post infection. Individual data with median values are shown. Dotted line represents the detection limit. ^{∗} = p < 0.05; ^{∗∗} = p < 0.01 as determined via Mann-Whitney U test.

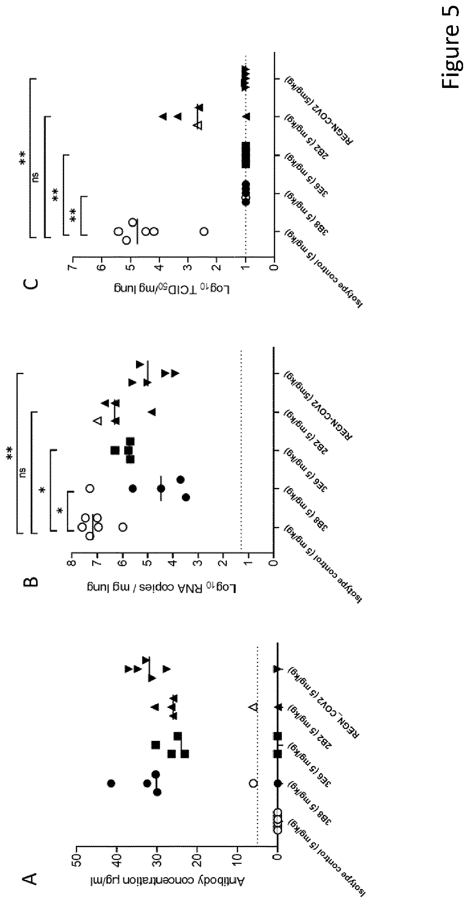

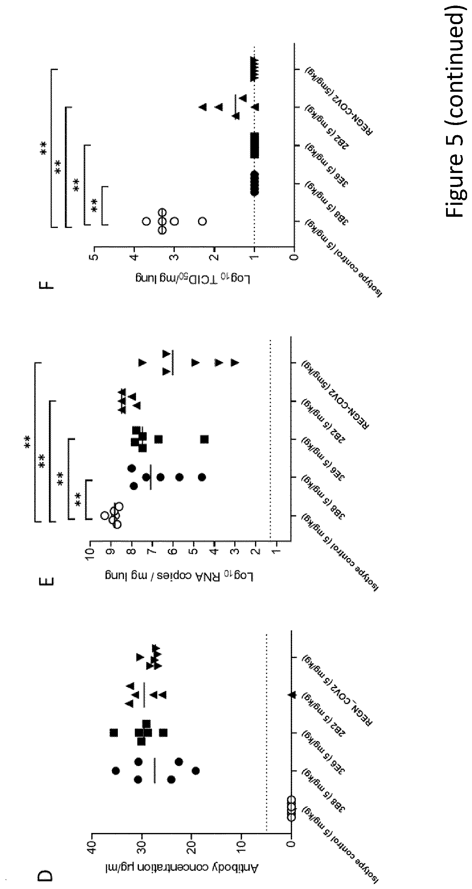

**Figure 5****: Treatment of SARS-CoV-2 beta and delta infection by antibodies 3B8, 3E6, 2B2 and REGN-COV-2 (5 mg/kg) in hamsters.** Concentration (µg/ml) of the administered antibodies at day 4 post infection in serum from hamsters infected with SARS-CoV-2 beta (A) or delta (D). Viral RNA levels in the lungs of hamsters infected with SARS-CoV-2 beta (B) or delta (E), on day 4 post infection. Infectious viral loads, in lungs of hamsters infected with SARS-CoV-2 beta (C) or delta (F), on day 4 post infection. Animals without detectable antibody serum levels were excluded from the graphs in panel B-C-E-F. Individual data and median values are shown. Dotted line represents the detection limit. ns = p > 0.05; ^{∗} = p < 0.05; ^{∗∗} = p < 0.01 as determined via Mann-Whitney U test.

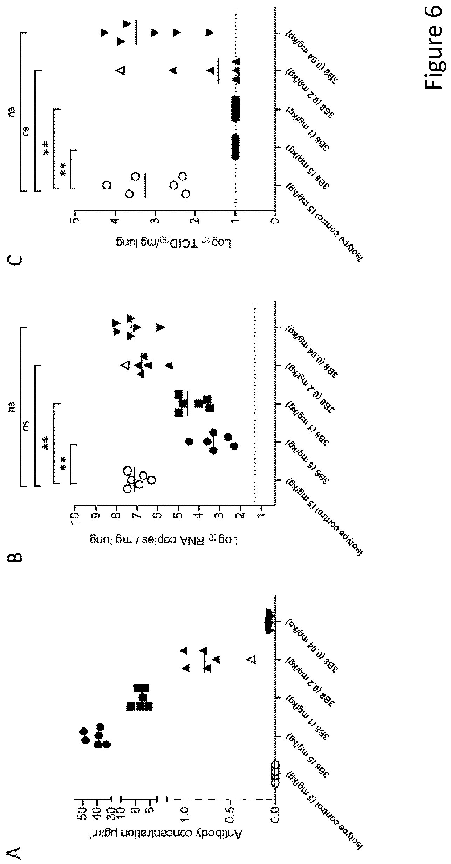

**Figure 6****: Dose-response experiment for treatment of SARS-CoV-2 delta infection with antibody 3B8 (5, 1, 0.2 or 0.04 mg/kg) in hamsters.** A) Concentration (µg/ml) of the administered antibodies in serum at day 4 post infection. B) Viral RNA levels, expressed as log₁₀ RNA copies per mg of lung tissue, on day 4 post infection. C) Infectious viral loads, expressed as log₁₀ TCID₅₀ per mg lung tissue, at day 4 post infection. Individual data and median values are shown. Dotted line represents the detection limit. ns = p > 0.05; ^{∗∗} = p < 0.01 as determined via Mann-Whitney U test.

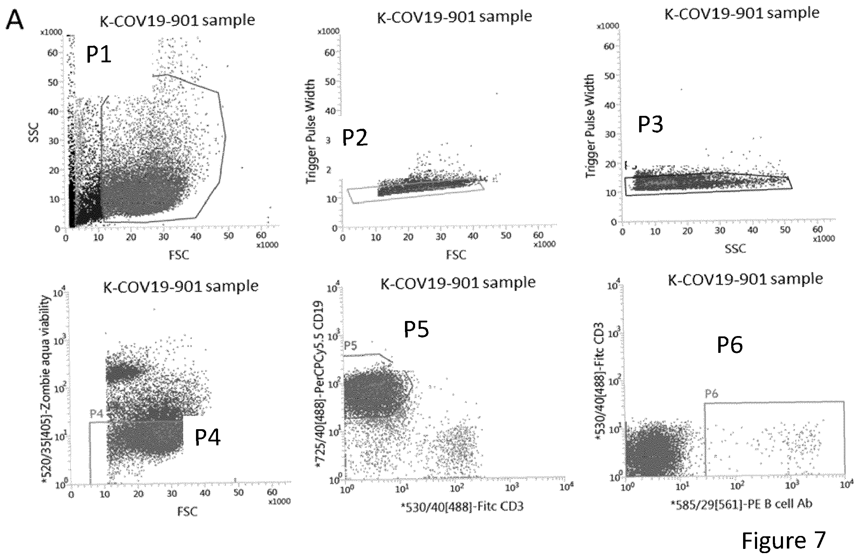

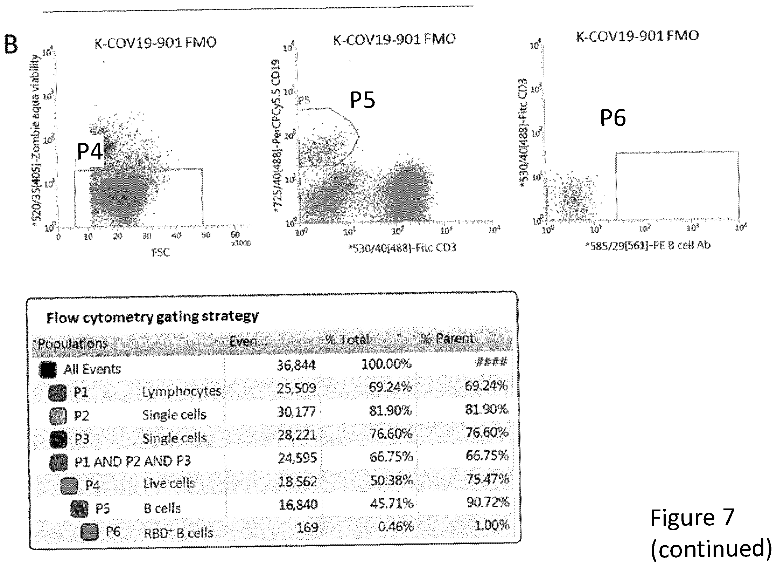

**Figure 7****: gating strategy for selection of RBD-specific B cells from PBMCs of patients K-COV19-901.**
   Lymphocytes were selected based on FSC and SSC, followed by selection of single cells and live cells (Zombia Aqua negative). Here, B cells were selected as CD19+ CD3- cells and evaluated for RBD surface staining using PE as fluorophore. Fully stained sample is shown in panel A, fluorescence minus one (FMO) control for RBD fluorescence is shown in panel B.

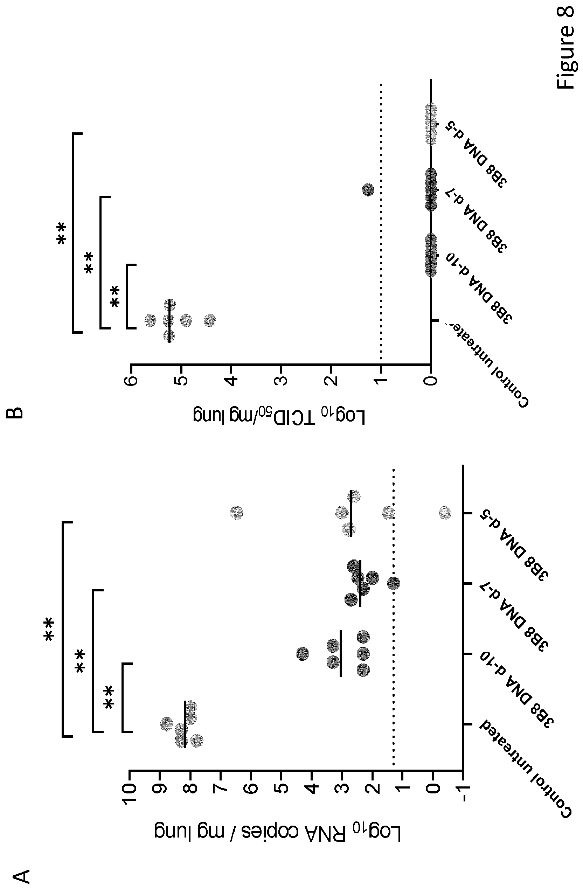

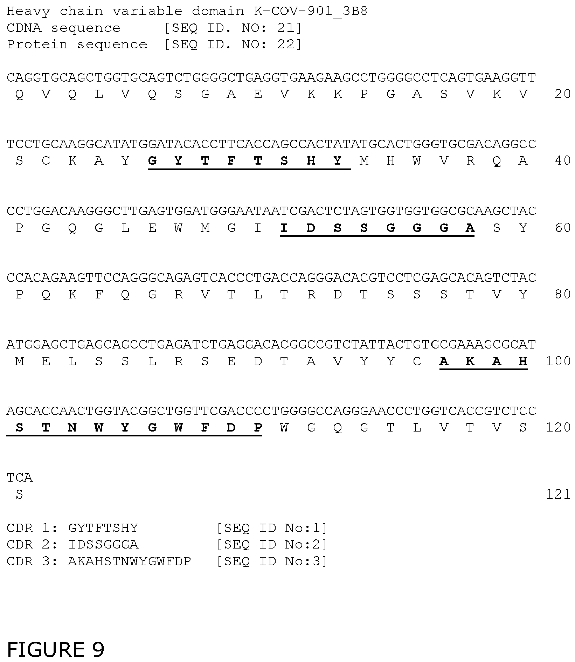

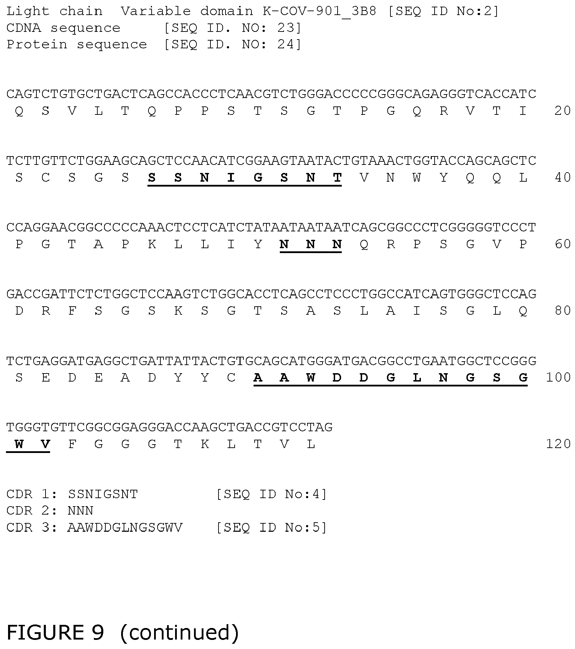

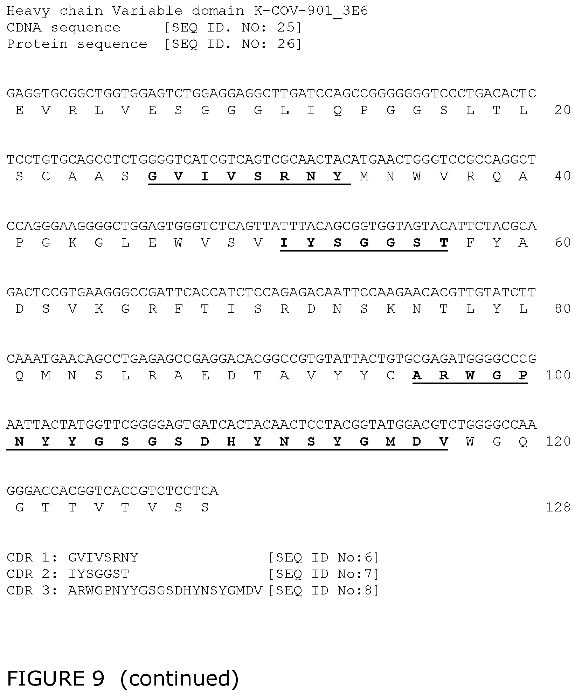

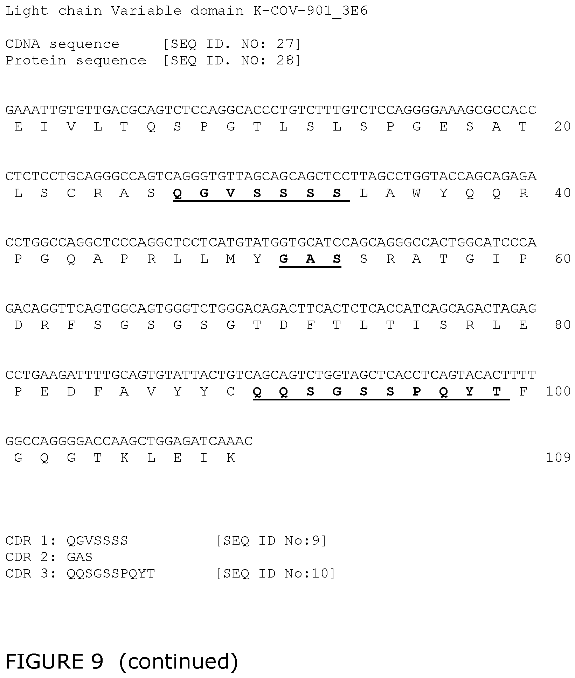

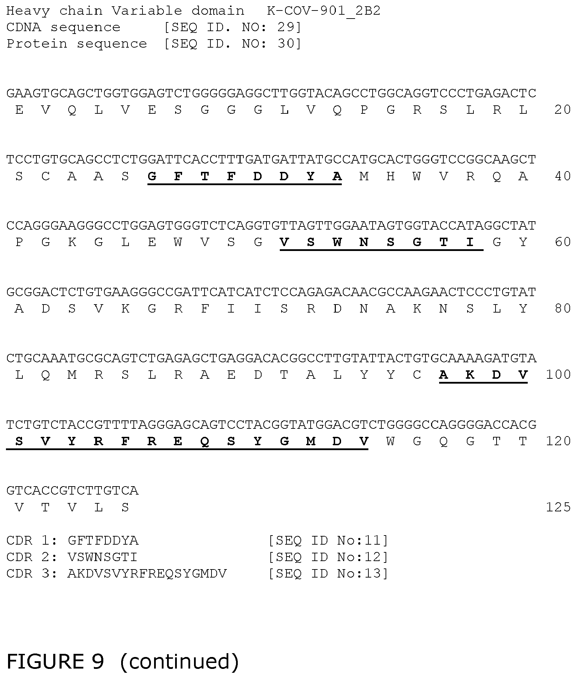

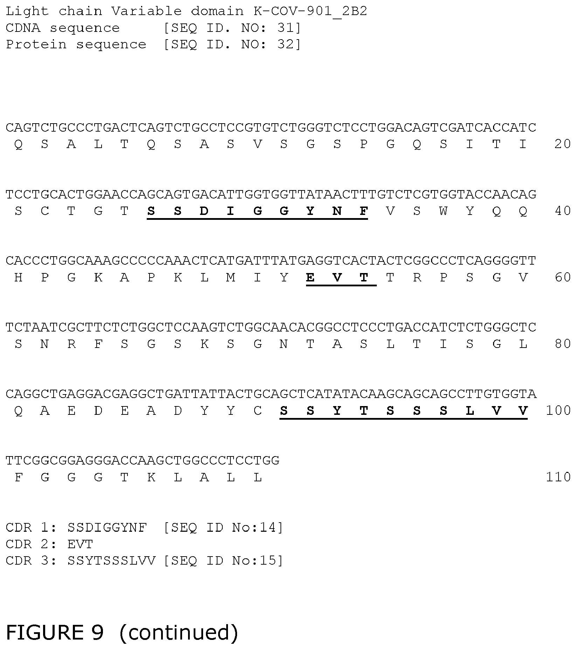

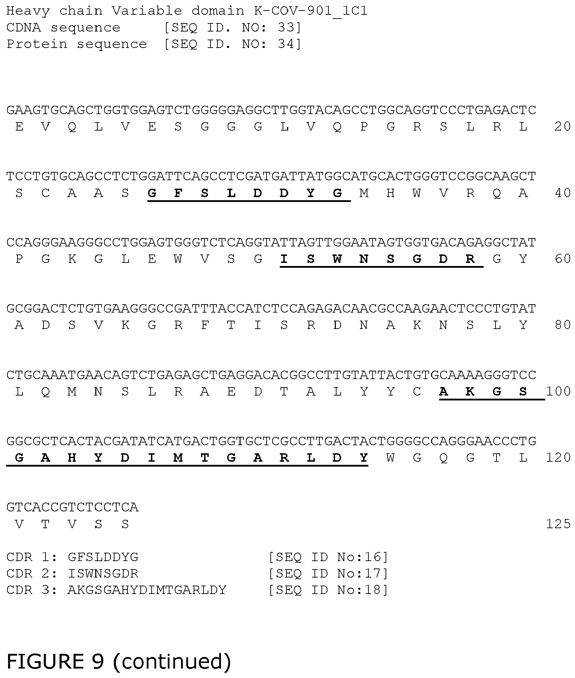

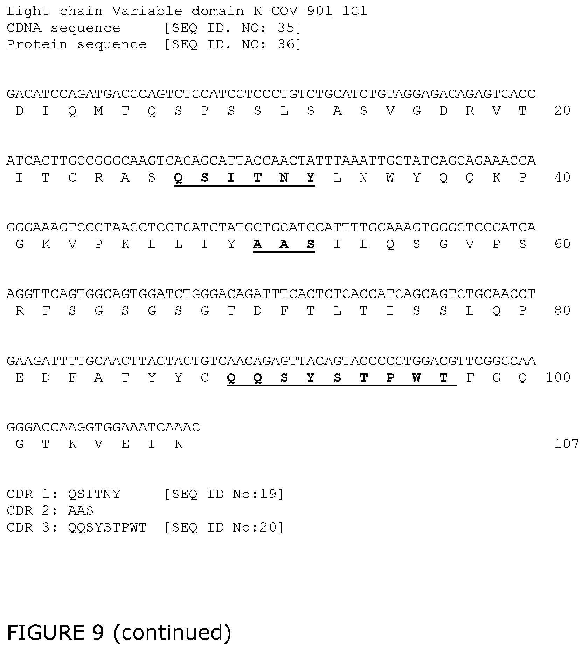

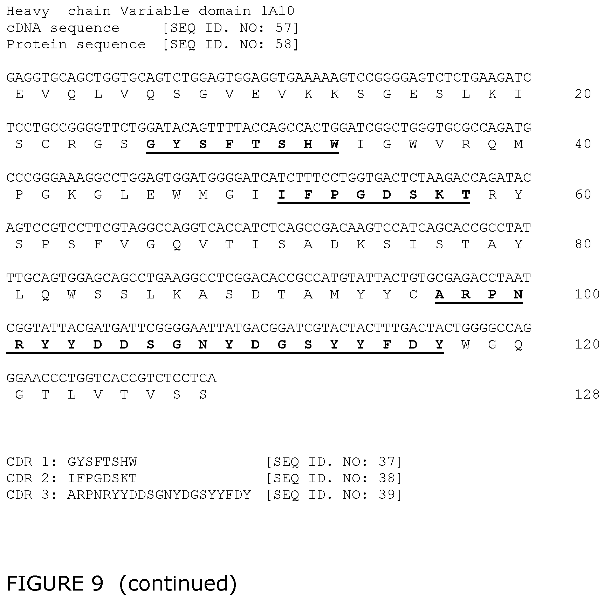

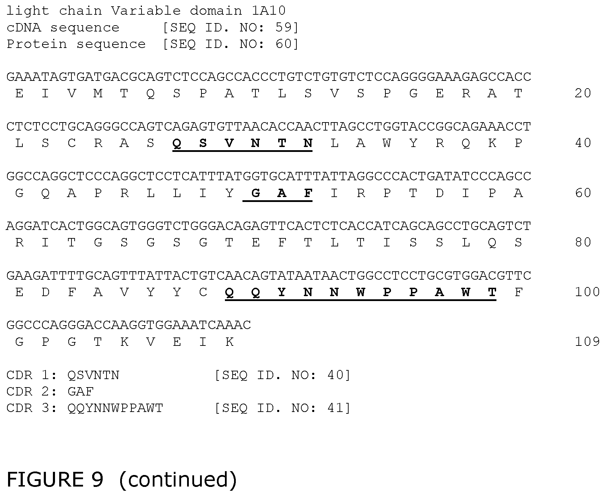

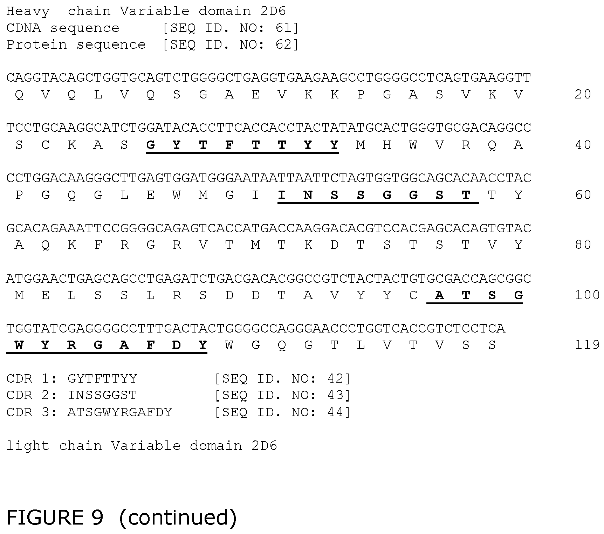

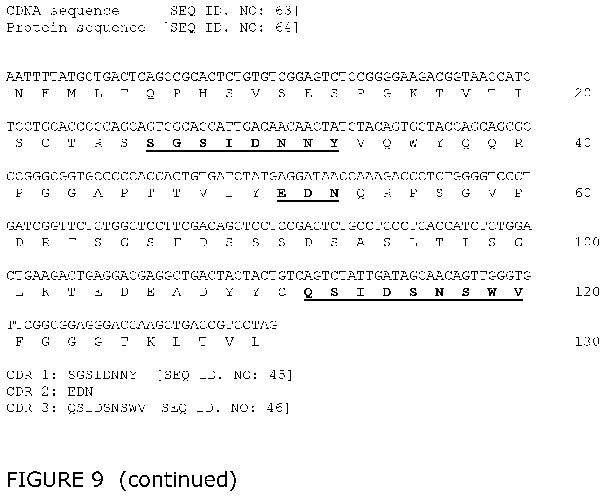

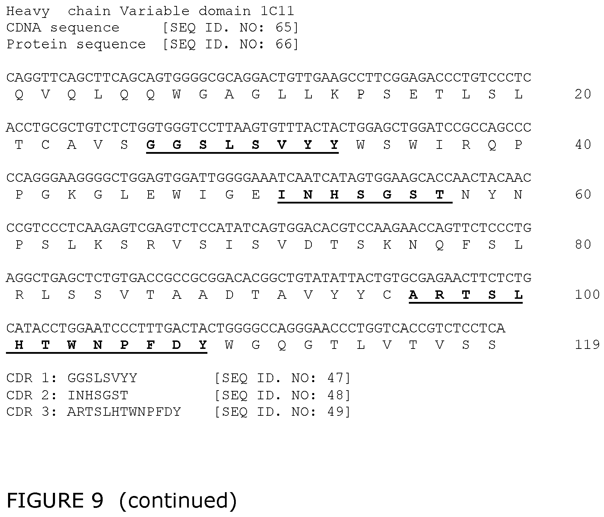

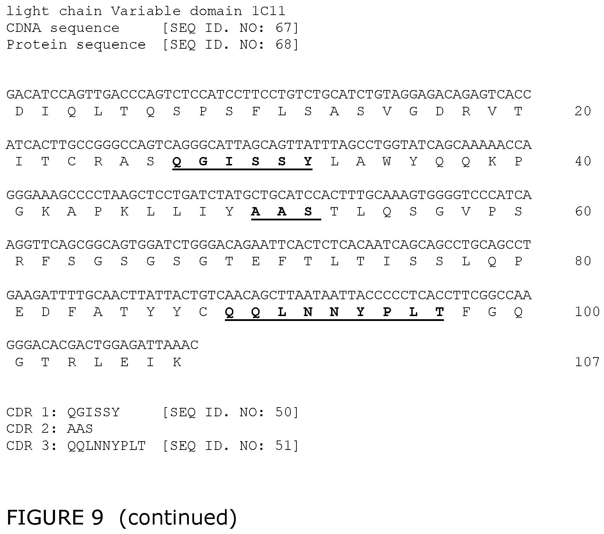

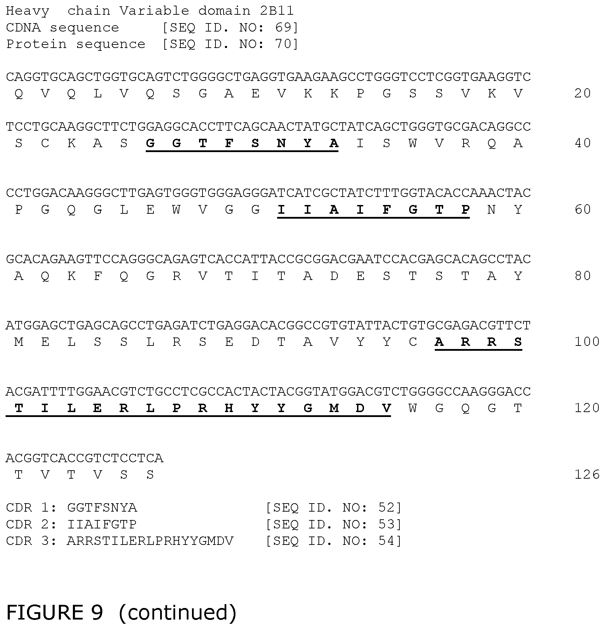

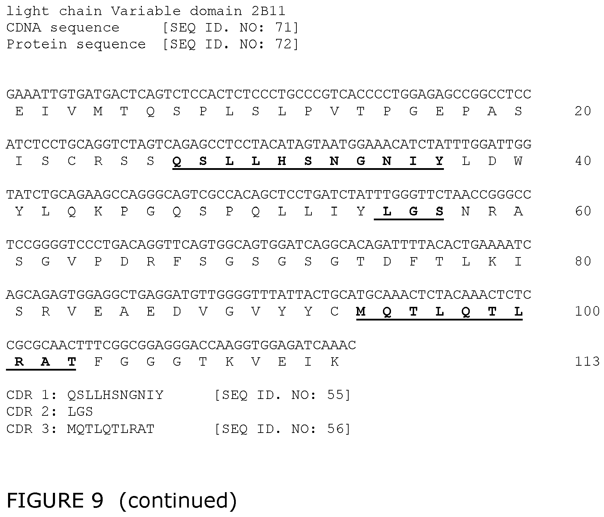

**Figure 8** **DNA base vaccination**
   Analysis of viral RNA levels (left panel) and infectious viral titers (right panel) upon injection of hamsters with 3B8 pDNA. Individual data with median values are shown. Dotted line represents the detection limit. ^{∗∗} = p < 0.01 as determined via Mann-Whitney U test.
**Figure 9**
   DNA and protein sequence of heavy and light chain variable regions.

Despite vaccination rates steadily increasing, less than 40% of individuals has been fully vaccinated worldwide and the number of SARS-CoV-2 related deaths continues to increase¹. In addition, vaccine-breakthrough infections have been reported and may be explained by the emergence of several SARS-CoV-2 variants showing a reduced sensitivity to vaccine-elicited antibody neutralization^{6,7,24-31}. Monoclonal antibodies may therefore play a crucial role to treat individuals that cannot be vaccinated, to treat vaccinated individuals still suffering from serious COVID-19 caused by infection with current or future SARS-CoV-2 variants of concern and to prevent rapid dissemination of viral infection in specific settings, such as elderly care homes.

In this study, 8 highly potent neutralizing antibodies (IC₅₀ < 1 nM) were identified from a convalescent COVID-19 patient and evaluated for their efficacy against different SARS-CoV-2 variants. Six out of eight antibodies bound all analyzed recombinant SARS-CoV-2 RBD antigens bearing single or multiple mutations. Affinities were evaluated for RBD antigens with single mutations and shown to be in the picomolar range. Corresponding to the antigen binding data, *in vitro* neutralization assays using live SARS-CoV-2 Wuhan, alpha, beta, gamma and delta strains showed that these antibodies potently cross-neutralized all variants with IC₅₀ values ranging from 0.01 to 3.97 nM. Interestingly, data from the cross-competition ELISA showed that these antibodies were not all targeting the same epitope, but could be divided into 4 epitope groups.

The most potent antibody of each of these epitope classes was tested for therapeutic activity *in vivo* in the Syrian golden hamster model. This model has been widely used to assess the efficacy of vaccines and therapeutics against SARS-CoV-2 infection as it closely mimics the clinical disease observed in humans³²⁻³⁴. Using this model, we designed three different therapeutic studies to evaluate the efficacy of our antibodies as a treatment for SARS-CoV-2 Wuhan, beta and delta infection. We showed that antibodies 3B8 and 3E6 reduced median viral RNA titers with a factor 50 - 5000 or 10 - 330 respectively when used as a treatment (5 mg/kg) for all tested SARS-CoV-2 strains. In addition, treatment with these antibodies resulted in undetectable levels of infectious virus in the lungs of almost all animals. Our *in vitro* and *in vivo* data suggest that 3B8 and 3E6 target an epitope conserved between the different variants, abolishing the need for combination therapy for the current SARS-CoV-2 variants. In addition, since we identified potent neutralizing antibodies from different epitope groups, combination therapy remains an option in the future if additional variants of concern appear.

Importantly, we also showed that antibody 3B8 is an ultrapotent therapeutic antibody, with treatment at a dose of 1 mg/kg resulting in undetectable infectious viral titers. Even when administered at 0.2 mg/kg, viral replication was reduced, with median infectious viral titers of the treatment group being 67 times lower compared to the isotype control group. This further highlights the potency of antibody 3B8 in a therapeutic setting^{2,34-38}. High potencies are crucial to decrease cost of goods, enable sustainable manufacturability and increase the number of doses produced annually, and may therefore also facilitate the availability of therapeutic mAbs to low- and middle-income countries.

When comparing the antibodies generated in this study with the commercially available ones, we noticed that K_{D} values observed for our antibodies were consistently between 3- and 200-fold lower than values reported for marketed antibodies of Vir Biotechnology, Regeneron Pharmaceuticals or Eli Lilly³⁹⁻⁴¹. However, when analyzing K_{D} values of REGN-COV2 antibodies 10933 and 10987 in our own experiments for benchmarking purposes, K_{D} values were similar to values observed for our mAbs (data not shown). In addition, it should be noted that antibodies 3B8 and 3E6 each performed equally well as monotherapy (when administered at 5 mg/kg) to treat SARS-CoV-2 infection beta and delta in hamsters when compared to the marketed REGN-COV2 antibody cocktail, which consists of two different antibodies²³. Moreover, we could observe therapeutic efficacy of antibody 3B8 *in vivo* at a dose of only 0.2 mg/kg. Interestingly, to the best of our knowledge, no *in vivo* therapeutic mAb efficacy data have been published yet for SARS-CoV-2 delta infection in hamsters. In addition, only for REGN-COV2 some therapeutic efficacy was reported in hamsters at such low mAb doses, although after infection of hamsters with SARS-CoV-2 Wuhan and using different readouts³².

Although no clear evidence for antibody-dependent disease enhancement (ADE) in COVID-19 patients has been reported until now, it has been suggested that its potential risk should be continuously monitored ^{4,8,34,42}. In this light, it should be emphasized that the high potency of 3B8 could be obtained without the contribution of Fc-mediated effector functions, as fully human IgG1 antibodies were not species-matched before administration to hamsters. This suggests that antibody 3B8 will tolerate the future introduction of Fc-effector silence mutations to avoid any potential risk of ADE, favoring its further evaluation as a future antibody treatment.

In conclusion, the high therapeutic potency of antibody 3B8 *in vivo* against SARS-CoV-2 delta, which is currently the most dominant variant worldwide, combined with its broad cross-reactivity against all other tested SARS-CoV-2 variants, make 3B8 a very interesting candidate to help fight the COVID pandemic.

The phrase "specifically bind(s)" or "bind(s) specifically" when referring to a peptide, refers to a peptide molecule which has intermediate or high binding affinity, exclusively or predominately, to a target molecule. The phrases "specifically binds to" refers to a binding reaction which is determinative of the presence of a target protein in the presence of a heterogeneous population of proteins and other biologics.

Thus, under designated assay conditions, the specified binding moieties bind preferentially to a particular target protein and do not bind in a significant amount to other components present in a test sample. Specific binding to a target protein under such conditions may require a binding moiety that is selected for its specificity for a particular target antigen. A variety of assay formats may be used to select ligands that are specifically reactive with a particular protein, for example, solid-phase ELISA immunoassays, or immunoprecipitation. Typically, a specific or selective reaction will be at least twice background signal or noise and more typically more than 10 times background. For instance, antibodies and antibody fragments of the invention preferentially bind to the RBD domain of the spike protein S1 subunit of Sars-Cov-2, whereby by "preferentially binding", "preferentially recognizing" or "preferentially reacting with" is meant that the antibodies or antibody fragments show greater binding capacity for RBD domain of the spike protein S1 subunit of Sars-Cov-2 as compared to any other antigen. The binding capacity of an antibody or antibody fragment to an antigen is reflective of its affinity and/or avidity for that antigen. Binding specificity can be expressed by association and dissociation constants as determined by ELISA or BIACORE.

The terms "antibody" and "antibodies" are recognized in the art and refer to proteins also known as immunoglobulins that bind to antigens. It is to be understood that these terms encompass conventional vertebrate antibodies like IgA, IgD, IgE, IgG, IgM, IgT, IgX and IgY, composed of at least two heavy and two light chains, as well as antibodies only composed of two heavy chains (VHH antibodies, IgNAR, heavy-chain antibodies, single-domain antibodies or nanobodies), and single-chain antibodies. In the case of conventional antibodies, the antigen binding sites are contributed to by the variable domains of both the heavy and light chains (VH and VL). The term "variable domain" refers to the part or domain of an antibody which is partially or fully responsible for antigen binding. Generally, variable domains will be amino acid sequences that essentially consist of 4 framework regions (FRI to FR4 respectively) and 3 complementarity determining regions (CDR1 to CDR3 respectively), or any suitable fragment of such an amino acid sequence which usually contains at least some of the amino acid residues that form at least one of the CDR's. Such variable domains and fragments are most preferably such that they comprise an immunoglobulin fold or are capable for forming, under suitable conditions, an immunoglobulin fold. Each CDR may contribute to a greater or lesser extent to antigen binding by the antibody. Single domain antibodies or heavy-chain antibodies can be found in camelids and sharks, and each of the antigen-binding sites of these antibodies is formed by a single heavy chain variable domain (VHH) only. Therefore, only three CDRs contribute to a greater or lesser extent to each antigen-binding site. Single chain antibodies (scFv) are derived from conventional antibodies by translational fusion of the VH and VL domains, separated by a flexible linker, into a single antigen-binding domain. Framework sequences of an antibody may be altered without altering the antigenic specificity of the antibody, or in order to change the binding affinity of the antibody. Furthermore, conventional antibodies may switch classes or isotypes without substantially affecting antigen-binding characteristics.

The term "complementarity determining region" or "CDR" refers to variable regions of either H (heavy) or L (light) chains (abbreviated as VH and VL, respectively) and contains the amino acid sequences capable of specifically binding to antigenic targets. These CDR regions account for the specificity of the antibody for a particular antigenic determinant structure. Such regions are also referred to as "hypervariable regions." The CDRs represent non-contiguous stretches of amino acids within the variable regions but, regardless of species, the positional locations of these critical amino acid sequences within the variable heavy and light chain regions have been found to have similar locations within the amino acid sequences of the variable chains. The accepted CDR regions and variable domains of an antibody are known to the skilled person and have been described by (Kabat EA et al. (1991) Sequences of proteins of immunological interest, 5th edn. U.S. Department of Health and Human Services, National Institutes for Health, Bethesda, MD) and (Padlan et al., (1995) Identification of specificity-determining residues in antibodies. FASEB J. 9, 133-139).

The skilled person is familiar with the concept that, upon alignment of corresponding CDRs of different antibodies with similar antigen specificity, the positions in the alignment which are conserved, i.e. identical in all sequences in the alignment, are critical for the antigen specificity of the antibodies. The residues of a particular CDR at these critical positions are known as "specificity-determining residues" or "SDRs". As a consequence, positions which are not conserved contribute less to the specificity of the antibodies and can be substituted without substantially affecting the antigen specificity of an antibody. Therefore, the skilled person is able to determine which residues could be substituted without substantially affecting antigen specificity of the antibody or antibody fragment. In the same way, the skilled person is able to determine the minimum sequence identity between a particular CDR of an antibody and the corresponding CDR of an antibody of the present invention, which is required for the particular CDR to have a similar antigen specificity as the corresponding CDR of an antibody of the present invention. The same holds true for the variable regions.

By the term "antibody fragment" is meant a fragment of an antibody that largely retains antigen-binding capacity of the antibody from which it is derived. Therefore, an antibody fragment of the invention is capable of preferentially binding to RBD domain of the spike protein S1 subunit of Sars-Cov-2. Antigen-binding capacity is determined by the variable domain or domains, more particularly by 1, 2, 3, 4, 5 or 6 CDRs located in the VH and/or VL domains in the case of conventional and single-chain antibodies, and 1, 2 or 3 CDRs in the case of single-domain antibodies. Preferred antibody fragments of the invention therefore comprise antigen-binding sites comprising 1, 2, 3, 4, 5 or 6 CDRs. Two or more CDRs may be physically separated from each other by connecting regions to provide a framework structure for the CDRs. More preferred antibody fragments of the invention comprise antigen-binding sites comprising 1 or 2 variable domains. Examples of antibody fragments are well-known to the skilled person and include the monovalent antigen-binding fragments (Fab), bivalent F(ab')2 fragments, Fv fragments (e.g. single chain antibodies scFv), miniaturized antibodies, single-domain antibody fragments like nanobodies (Nelson AL 2010, Antibody fragments: hope and hype. mAbs 2:77-83). Antibody fragments of the invention may be obtained by enzymatic or chemical proteolysis, or by recombinant DNA technology techniques well known to the skilled person.

Antibodies and antibody fragments of the invention may be further chemically conjugated, non-covalently bound, or translationally fused to other proteins. Single chain antibodies scFv are an example of translational fusion between a VH and a VL domain. Further examples are albumin-conjugated antibodies or antibody fragments, bivalent diabodies, and monospecific and bispecific tandem svFcs (Nelson AL 2010, Antibody fragments: hope and hype. mAbs 2:77-83).

A single-chain antibody (also referred to as "scFv") can be prepared by linking a heavy chain V region and a light chain V region of an antibody (for a review of scFv see Pluckthun "The Pharmacology of Monoclonal Antibodies" Vol. 113, eds. Rosenburg and Moore, Springer Verlag, New York, pp.269-315 (1994)). Methods for preparing single-chain antibodies are known in the art (see, for example, US Patent Nos. 4,946,778, 5,260,203, 5,091,513, and 5,455,030). In such scFvs, the heavy chain V region and the light chain V region are linked together via a linker, preferably, a polypeptide linker (Huston, J. S. et al., Proc. Natl. Acad. Sci. U.S.A, 1988, 85, 5879-5883). The heavy chain V region and the light chain V region in a scFv may be derived from the same antibody, or from different antibodies.

An "Fv" fragment is the smallest antibody fragment, and contains a complete antigen recognition site and a binding site. This region is a dimer (VH-VL dimer) wherein the variable regions of each of the heavy chain and light chain are strongly connected by a noncovalent bond. The three CDRs of each of the variable regions interact with each other to form an antigen binding site on the surface of the VH-VL dimer. In other words, a total of six CDRs from the heavy and light chains function together as an antibody's antigen-binding site. However, a variable region (or a half Fv, which contains only three antigen-specific CDRs) alone is also known to be able to recognize and bind to an antigen, although its affinity is lower than the affinity of the entire binding site. Thus, a preferred antibody fragment of the present invention is an Fv fragment, but is not limited thereto.

Such an antibody fragment may be a polypeptide which comprises an antibody fragment of heavy or light chain CDRs which are conserved, and which can recognize and bind its antigen.

A Fab fragment (also referred to as F(ab)) also contains a light chain constant region and heavy chain constant region (CH1). For example, papain digestion of an antibody produces the two kinds of fragments: an antigen-binding fragment, called a Fab fragment, containing the variable regions of a heavy chain and light chain, which serve as a single antigen-binding domain; and the remaining portion, which is called an "Fc" because it is readily crystallized. A Fab' fragment is different from a Fab fragment in that a Fab' fragment also has several residues derived from the carboxyl terminus of a heavy chain CH1 region, which contains one or more cysteine residues from the hinge region of an antibody.

A Fab' fragment is, however, structurally equivalent to Fab in that both are antigen-binding fragments which comprise the variable regions of a heavy chain and light chain, which serve as a single antigen-binding domain. Herein, an antigen-binding fragment comprising the variable regions of a heavy chain and light chain which serve as a single antigen-binding domain, and which is equivalent to that obtained by papain digestion, is referred to as a "Fab-like antibody", even when it is not identical to an antibody fragment produced by protease digestion. Fab'-SH is Fab' with one or more cysteine residues having free thiol groups in its constant region. A F(ab') fragment is produced by cleaving the disulfide bond between the cysteine residues in the hinge region of F(ab')2. Other chemically crosslinked antibody fragments are also known to those skilled in the art.

Pepsin digestion of an antibody yields two fragments; one is a F(ab')2 fragment which comprises two antigen-binding domains and can cross-react with antigens, and the other is the remaining fragment (referred to as pFc'). Herein, an antibody fragment equivalent to that obtained by pepsin digestion is referred to as a "F(ab')2-like antibody" when it comprises two antigen-binding domains and can cross-react with antigens. Such antibody fragments can also be produced, for example, by genetic engineering. Such antibody fragments can also be isolated, for example, from the antibody phage library described above. Alternatively, F(ab')2-SH fragments can be recovered directly from hosts, such as E. coli, and then allowed to form F(ab')2 fragments by chemical crosslinking (Carter et al., Bio/Technology 10:163-167 (1992)).

Antibodies and antibody fragments of the invention may be further modified. Examples of such modifications include the addition of detectable enzymatic, fluorescent, luminescent, or radioactive marker groups or molecules that act in detection such as streptavidin. Other examples include the chemical modification to alter the half-life of antibodies and antibody fragments, such as PEGylation. Still other examples add effector moieties to antibodies and antibody fragments, such as toxins, radioisotopes, enzymes, cytokines, and antigens (Nelson AL 2010, Antibody fragments: hope and hype. mAbs 2:77-83).

Antibodies or antibody fragments may be further modified into an antibody-derived scaffold or antibody-like scaffolds that largely retains antigen-binding capacity of the antibody or antibody fragments from which it is derived. Examples of antibody-derived scaffolds or antibody-like scaffolds are domain antibodies (dAb) that selectively or preferentially bind the same epitope as a natural antibody, for instance dAb with fully human frameworks, for instance dAb fused to a human Fc domain or for instance nanobodies engineered in a molecule that has an IgG-like circulating half-life in humans or antibody fragments with retained antigen-binding capacity or domain antibody with active scaffolds for controlled and cell delivery.

Single domain antibodies can be engineered into antibody like fragments. Single domain antibodies are antibodies whose complementary determining regions are part of a single domain polypeptide. Examples include, but are not limited to, heavy chain antibodies, antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, goat, rabbit, bovine. According to one aspect of the invention, a single domain antibody as used herein is a naturally occurring single domain antibody known as heavy chain antibody devoid of light chains. Such single domain antibodies are disclosed in WO9404678 for example. For clarity reasons, this variable domain derived from a heavy chain antibody naturally devoid of light chain is known herein as a VHH or nanobody to distinguish it from the conventional VH of four chain immunoglobulins. Such a VHH molecule can be derived from antibodies raised in Camelidae species, for example in camel, llama, dromedary, alpaca and guanaco or Elasmobranchii species for instance skates, rays (batoidea), and sharks (selachii). Other species besides Camelidae may produce heavy chain antibodies naturally devoid of light chain; such VHHs are within the scope of the invention. The single-chain polypeptide may be produced by various methods well known in the art such as genetic engineering technique and chemical synthesis. The genetic engineering technique includes constructing a replicable cloning vector or expression vector, transforming the host cell with the vector, culturing the transformed host cell to express the nucleic acid in it, collecting and purifying the single-chain polypeptide. The vector usually comprises the nucleic acid encoding one of the two single-chain polypeptides constituting the diabody-type bispecific antibody according to the present invention. In such case, the resulting two kinds of the vectors are preferably introduced into the same host cell.

Alternatively, the two kinds of nucleic acid encoding the different single-chain polypeptides from each other may be comprised in the same vector. The term "replicable expression vector" or "expression vector" as used herein refers to a piece of DNA (usually double-stranded) that may comprise a fragment of a foreign DNA fragment inserted therein. The foreign DNA is also defined as a "heterologous DNA", which cannot be found naturally in a host cell in interest. The vector is used to carry or convey the foreign or heterologous DNA into an appropriate host cell. Once the vector is introduced into the host cell, it may be replicated independently from a chromosomal DNA of the host cell to produce copies of the vector and foreign DNA inserted therein. The vector also comprises elements essential for translating the foreign DNA into a polypeptide so that the polypeptide molecules encoded by the foreign DNA will be synthesized very quickly.

The above vector means a DNA construct comprising an appropriate control sequence and DNA sequence that are operably linked together (i.e., linked together so that the foreign DNA can be expressed). The control sequence includes a promoter for transcription, an optional operator sequence to regulate the transcription, a sequence encoding an appropriate mRNA ribosome-biding site, an enhancer, a polyadenylation sequence, and a sequence controlling the termination of transcription and translation. The vector may further comprise various sequences known in the art, such as a restriction enzyme cleaving site, a marker gene (selection gene) such as a drug-resistant gene, a signal sequence, and a leader sequence. These sequences and elements may be optionally selected by those skilled in the art depending on the kinds of the foreign DNA and host cell, and conditions of culture medium.

The vector may be in any form such as a plasmid, phage particle, or just simply genomic insert. Once the appropriate host cell is transformed with the vector, the vector will be replicated or function independently from the genome of the host cell, or the vector will alternatively be integrated into the genome of the cell. Any cell known in the art may be used as the host cell, for example, there may be mentioned procaryotic cells such as including E. coli, eucaryotic cells such as mammalian cells such Chinese hamster ovary (CHO) cell and human cells, yeast, and insect cells.

Although the single-chain polypeptide obtained by the expression in the host cell is usually secreted and collected from the culture medium, it may be also collected from cell lysate when it is directly expressed without a secretion signal. In case the single-chain polypeptide has a membrane-binding property, it may be released from the membrane with an appropriate surfactant such as Triton-X100.

Purification of the polypeptide may be carried out by any method known to those skilled in the art such as centrifugation, hydroxyapatite chromatography, gel electrophoresis, dialysis, separation on ion-exchange chromatography, ethanol precipitation, reverse phase HPLC, silica chromatography, heparin-sepharose chromatography, anion-or cation-resin chromatography such as polyaspartic acid column, chromato-focusing, SDS-PAGE, precipitation with ammonium sulfate, and affinity chromatography. The affinity chromatography, which utilizes affinity with a peptide tag of the single-chain polypeptide, is one of the preferred purification techniques with a high efficiency.

Since the collected single-chain polypeptide may be often included in an insoluble fraction, the polypeptide is preferably purified after being solubilized and denatured. The solubilization treatment may be carried out with the use of any agent known in the art, including alcohol such ethanol, a dissolving agent such as guanidine hydrochloride and urea.

The antibody-like fragments according to the present invention are produced by assembling the single-chain polypeptides, eventually on a scaffold, and separating and collecting the thus formed antibody-like fragments.

Assembling treatment brings the single-chain polypeptides back in an appropriate spatial arrangement in which a desired biological activity is shown. Thus, since this treatment brings the polypeptides or domains back into an assembling state, it may be considered "re-assembling." It may be also called "re-constitution" or "refolding" in view of gaining the desired biological activity.

The assembling treatment may be carried out by any method known in the art preferably by gradually lowering the concentration of a denaturing agent such as guanidine hydrochloride in a solution comprising the single-chain polypeptide by means of dialysis. During these processes, an anti-coagulant or oxidizing agent may be optionally added in a reaction system in order to promote the oxidation. The separation and collection of the formed antibody-like fragment may be done by any method known in the art as well.

VHHs, according to the present invention, and as known to the skilled addressee, are heavy chain variable domains derived from immunoglobulins naturally devoid of light chains such as those derived from Camelidae as described in WO9404678 (and referred to hereinafter as VHH domains or nanobodies). VHH molecules are about 10× smaller than IgG molecules. They are single polypeptides and very stable, resisting extreme pH and temperature conditions. Moreover, they are resistant to the action of proteases which is not the case for conventional antibodies. Furthermore, in vitro expression of VHHs produces high yield, properly folded functional VHHs. In addition, antibodies generated in Camelids will recognize epitopes other than those recognised by antibodies generated in vitro through the use of antibody libraries or via immunization of mammals other than Camelids or Elasmobranchii species (WO 9749805). As such, anti-albumin VHH's may interact in a more efficient way with serum albumin which is known to be a carrier protein. As a carrier protein some of the epitopes of serum albumin may be inaccessible by bound proteins, peptides and small chemical compounds. Since VHH's are known to bind into 'unusual' or nonconventional epitopes such as cavities (WO9749805), the affinity of such VHH's to circulating albumin may be increased.

In one embodiment, the antibodies and antibody fragments of the invention are humanized. Antibody fragments derived from an antibody of the invention can be fused to the Fc region of a human antibody, in order to obtain humanized antibodies and antibody fragments. Humanized antibodies or antibody fragments can also be obtained by grafting of one or more CDRs or only their specificity-determining residues (SDRs), optionally together with one or more framework residues important for optimal CDR functionality, of a non-human antibody having the desired antigen-binding specificity, into framework polypeptide sequences of a human antibody or antibody fragment, or even into a universal humanized nanobody scaffold. Methods to humanize antibodies are well known to those skilled in the art (see e.g. (De Pascalis et al., J Immunol. 169:6, 3076-3084 (2002); Kashmiri et al., Methods, 36:25-34 (2005); Almagro and Fransson, Front.Biosci., 13:1619-1633 (2008); Vincke, et al., J Biol Chem., 284:3273-3284 (2009); Borras et al. J Biol Chem. 19(285):9054-9066 (2010); Harding et al., Mabs, 2:256-265 (2010)).

The term "humanized antibody" as used herein means a human immunoglobulin (a recipient antibody) in which at least part of the residues of complementary-determining region (CDR) is replaced with residues derived from the CDR of a non-human animal antibody (a donor antibody) that has a desired specificity, affinity and capability, such as those of mouse, rat, and rabbit. In some cases, the residue(s) of a Fv framework (FR) in the human immunoglobulin is replaced with residue(s) of the corresponding non-human antibody. The humanized antibody may further comprise a residue that is not found in the recipient antibody or the introduced CDR or framework. These changes are made in order to optimize or improve the properties of the resulting antibody. More detailed information on these changes are referred to Jones et al., Nature, 321:522-525 (1986); Reichmann et al., Nature, 332:323-329 (1988); EP0239400B; Presta, Curr. Op. Struct. Biol, 2:593-596 (1992); and EP0451216B.

The antibody fragments of the present invention can also be generated using various phage display methods known in the art. In phage display methods, functional antibody domains are displayed on the surface of phage particles which carry the polynucleotide sequences encoding them. In particular, such phage can be utilized to display epitope-binding domains expressed from a repertoire or combinatorial antibody library (e.g., human or murine). Phage expressing an epitope-binding domain that binds the antigen of interest can be selected or identified with antigen, e.g., using labeled antigen bound or captured to a solid surface or bead. Phages used in these methods are typically filamentous phage including fd and M13 binding domains expressed from phage with antigen-binding antibody domains recombinantly fused to either the phage gene III or gene VIII protein. Examples of phage display methods that can be used to make the antibodies or antibody fragments of the present invention include those disclosed in (Kettleborough et al Eur. J. Immunol., 24:952-958 (1994); Burton et al., Advances in Immunology, 57: 191-280 (1994); Brinkman et al., J. Immunol. Methods, 182: 41-50 (1995); Ames et al., J. Immunol. Methods, 184: 177-186 (1995); Persic et al., Gene, 187: 9-18 (1997); W0/1992/001047; WO 5 90102809; WO 91/10737; WO 92/01047; WO 92/18619; WO 93/11236; WO 95/15982; WO 95/20401; and US Patents 5,698,426; 15 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108).

After phage selection, the regions of the phage encoding the fragments can be isolated and used to generate the epitope-binding fragments through expression in a chosen host, including mammalian cells, insect cells, plant cells, yeast, and bacteria, using recombinant DNA technology. For example, techniques to recombinantly produce antigen-binding fragments can also be employed using methods known in the art such as those disclosed in WO 92/22324; Better et al., Science, 240: 1041-1104 (1988); Mullinax et al., Biotechniques, 12(6):864-869 (1992); Sawai et al., AJRI, 34:2634 (1995). Examples of techniques which can be used to produce single-chain Fvs and antibodies include those described in U.S. Pat. Nos. 4,946,778 and 5,258,498; Skerra et al., Science, 240:1038-1040 (1988); Huston et al., Methods in Enzymology, 203:46-88 (1991); Shu et al., PNAS, 90:7995-7999 (1993).

Changes may be made to the residues that comprise the CDRs without interfering with the ability of the antibody to recognize and bind its cognate epitope. For example, changes that do not affect epitope recognition, yet increase the binding affinity of the antibody for the epitope may be made. Several studies have surveyed the effects of introducing one or more amino acid changes at various positions in the sequence of an antibody, based on the knowledge of the primary antibody sequence, on its properties such as binding and level of expression (Yang et al., J Mol Biol. 254[3]:392-403 (1995); Vaughan et al., Nat Biotechnol. 16[6]: 535-539 (1998); Rader et al., Proc Natl Acad Sci U.S.A. 95[15]:8910-8915 (1998)). In these studies (so called affinity maturation techniques), altered versions of the antibody have been generated by changing the sequences of the encoding genes in the CDR1, CDR2, CDR3, or framework regions, using methods such as oligonucleotide-mediated site-directed mutagenesis, cassette mutagenesis, error-prone PCR, DNA shuffling, or mutator-strains of E. coli (Vaughan et al., Nat Biotechnol. 16[6]:535-539 (1998)). These methods of changing the sequence of the antibody have resulted in improved affinities of the resulting antibodies (Gram et al., Proc Natl Acad Sci U.S.A. 89[8]:3576-3580 (1992); Davies and Riechmann, Immunotechnology, 2[3]:169-179 (1996); Thompson et al., J Mol Biol. 256[1]:77-88 (1996); Boder et al., Proc Natl Acad Sci U.S.A. 97[20]:10701-10705 (2000); Furukawa et al., J Biol Chem. 276[29]:27622-27628 (2001); Short et al., J Biol Chem., 277[19]:16365-16370 (2002)).

In a preferred embodiment of the invention, the antibody of the invention is monoclonal. The term "monoclonal antibody" is well recognized in the art and refers to an antibody or a homogenous population of antibodies that is derived from a single clone. Individual antibodies from a monoclonal antibody population are essentially identical, in that minor naturally occurring mutations may be present. Antibodies from a monoclonal antibody population show a homogenous binding specificity and affinity for a particular epitope.

As used herein, "percentage identity" or"% sequence identity" between two or more amino acid sequences or two or more nucleotide sequences refers to the ratio, expressed in %, of: the number of amino acids or nucleotides in an optimal alignment of the amino acid sequences or nucleotide sequences that are identical in both sequences (i.e. match), to the length of the alignment, i.e. the number of aligned positions, including gaps if any.

The term "nucleic acid" is intended to include DNA molecules and RNA molecules. A nucleic acid can be single-stranded or double-stranded.

The nucleic acids of the invention are present in whole cells, in a cell lysate, or in a partially purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCI banding, column chromatography, agarose gel electrophoresis and others well known in the art. see, e.g., Sambrook, Tijssen and Ausubel. The nucleic acid sequences of the invention and other nucleic acids used to practice this invention, whether RNA, cDNA, genomic DNA, or hybrids thereof, may be isolated from a variety of sources, genetically engineered, amplified, and/or expressed recombinantly. Any recombinant expression system can be used, including, in addition to bacterial, e.g., yeast, insect or mammalian systems. Alternatively, these nucleic acids can be chemically synthesized in vitro. Techniques for the manipulation of nucleic acids, such as, e.g., subcloning into expression vectors, labeling probes, sequencing, and hybridization are well described in the scientific and patent literature, see, e.g., Sambrook, Tijssen and Ausubel. Nucleic acids can be analyzed and quantified by any of a number of general means well known to those of skill in the art. These include, e.g., analytical biochemical methods such as NMR, spectrophotometry, radiography, electrophoresis, capillary electrophoresis, high performance liquid chromatography (HPLC), thin layer chromatography (TLC), and hyperdiffusion chromatography, various immunological methods, such as fluid or gel precipition reactions, immunodiffusion (single or double), immunoelectrophoresis, radioimmunoassays (RIAs), enzyme-linked immunosorbent assays (ELISAs), immunofluorescent assays, Southern analysis, Northern analysis, dot-blot analysis, gel electrophoresis (e.g., SDS-PAGE), RT-PCR, quantitative PCR, other nucleic acid or target or signal amplification methods, radiolabeling, scintillation counting, and affinity chromatography.

The nucleic acid compositions of the present invention, while often in a native sequence (except for modified restriction sites and the like), from either cDNA, genomic or mixtures may be mutated, thereof in accordance with standard techniques to provide gene sequences. For coding sequences, these mutations, may affect amino acid sequence as desired. In particular, DNA sequences substantially homologous to or derived from such sequences described herein are contemplated (where "derived" indicates that a sequence is identical or modified from another sequence).

### EXAMPLES

### EXAMPLE 1. Identification and characterization of anti-SARS-CoV-2 antibodies from convalescent COVID-19 patients

To identify fully human SARS-CoV-2 neutralizing antibodies, we first analyzed RBD-binding titers and neutralizing antibody titers in serum, as well as the percentage RBD-positive B cells in PBMCs from 26 convalescent COVID-19 patients (see Table 1).

**Table 1: overview of RBD-binding titers and neutralizing antibody titers in serum, and percentage RBD-positive B cells in PBMCs from 26 convalescent COVID-19 patients**

| **Patient ID** | **anti-RBD Ab (µg/ml)** | **Neutralizing Abs (SNT50 titer)** | **% RBD-positive of B cells** |
|---|---|---|---|
| K-COV19-901 | 182,16 | 1209 | 0,80% |
| K-COV19-902 | 21,65 | 96 | 0,00% |
| K-COV19-006 | 387,59 | 7022 | 0,00% |
| K-COV19-007 | 5,98 | / | 0,00% |
| K-COV19-009 | 7,57 | 8 | 0,04% |
| K-COV19-010 | 15,58 | 74 | 0,03% |
| K-COV19-019 | 26,07 | 771 | 0,05% |
| K-COV19-021 | 30,80 | 506 | 0,19% |
| K-COV19-028 | 25,75 | 493 | 0,00% |
| K-COV19-034 | 48,82 | 810 | 0,02% |
| K-COV19-041 | 8,66 | 201 | 0,09% |
| K-COV19-044 | 40,22 | 876 | 0,00% |
| L-COV19-001 | 14,75 | 178 | 0,14% |
| L-COV19-002 | 24,37 | 248 | 0,30% |
| L-COV19-003 | 51,37 | 481 | 0,18% |
| L-COV19-004 | 48,71 | 302 | 0,07% |
| L-COV19-005 | 196,52 | 2123 | 0,14% |
| L-COV19-007 | 32,00 | 829 | 0,22% |
| L-COV19-008 | 54,97 | 1539 | 0,35% |
| L-COV19-009 | 32,07 | 1199 | 0,21% |
| L-COV19-010 | 16,76 | 104 | 0,19% |
| L-COV19-011 | 32,26 | 495 | 0,11% |
| L-COV19-012 | 13,99 | 140 | 0,07% |
| L-COV19-015 | 41,20 | 3077 | 0,14% |
| L-COV19-018 | 10,36 | 69 | 0,11% |

Patient K-COV19-901, having both a high titer of neutralizing antibodies and a high number of RBD-specific B cells, was selected for the isolation of individual RBD-specific B cells via fluorescence-activated cell sorting (FACS), using biotinylated RBD (Wuhan isolate) combined with streptavidin-PE as bait (Figure 7). Next, these single B cells were used as starting material for our single B cell cloning strategy, where we amplified the coding sequence of the IgG antibody heavy and light chain variable domains and combined them with the human IgG1 constant domain in a vector for recombinant antibody production. A panel of 20 unique antibody sequences (labelled K-COV-901-X, further mentioned as "X") was selected for *in vitro* production and subsequent characterization. All 20 antibodies retained RBD and trimeric spike antigen (Wuhan) binding *in vitro* when analyzed via ELISA or surface plasmon resonance (SPR) assays. In addition, antibodies showed picomolar affinities to the RBD antigen and trimeric spike antigen with equilibrium constants (K_{D} values) ranging from 31 to 443 pM and 32 to 243 pM, respectively (Table 2).

Next, we evaluated the functional activity of these antibodies *in vitro.* To this end, we used a pseudovirus assay with vesicular stomatitis virus (VSV) expressing the SARS-CoV-2 spike protein (D614G) on its surface and evaluated pseudovirus infection of Vero E6 cells in the presence and absence of each of the antibodies. Out of 20 mAbs, 16 could neutralize SARS-CoV-2 pseudovirus infection, with 8 very potent mAbs having a half-maximal 50% inhibitory concentration (IC₅₀) lower than 1 nM and antibody 3B8 having an IC₅₀ of only 0.017 nM (Figure 1 & Table 2). Given their potent neutralizing capacity, these 8 mAbs were selected for further characterization.

**Table 2: Overview of antigen binding, affinity and neutralizing capacity of 20 selected antibodies.**

| | **ELISA antigen binding** | | **Affinity K_{D} (pM)** | | **Neutralization IC₅₀(nM)** |
|---|---|---|---|---|---|
| **Antibody** | **RBD (Wuhan)** | **Trimeric spike (Wuhan)** | **RBD (Wuhan)** | **Trimeric spike (Wuhan)** | **Trimeric spike (D614G)** |
| ***1A10*** | **+** | **+** | ***199* ± *58*** | ***89* ± *45*** | ***0.83*** |
| 1B11 | + | + | 443 ± 4 | 89 ± 18 | / |
| ***1C1*** | **+** | **+** | ***41* ± *12*** | ***44 ± 12*** | ***0.78*** |
| ***1C11*** | **+** | **+** | ***87* ± *24*** | ***84* ± *29*** | ***0.20*** |
| 1D5 | + | + | 111 ± 13 | 127 ± 78 | 15.74 |
| 1B5 | + | + | 45 ± 2 | 56 ± 16 | 1.83 |
| 2A2 | + | + | 220 ± 17 | 176 ± 88 | 27.53 |
| 2A8 | + | + | 78 ± 8 | 74 ± 5 | 8.91 |
| ***2B11*** | + | + | ***48 ± 21*** | ***32 ± 8*** | ***0.71*** |
| ***2B2*** | + | + | ***31 ± 7*** | ***63 ± 14*** | ***0.18*** |
| 2C8 | + | + | 64 ± 8 | 91 ± 15 | / |
| 2D10 | + | + | 52 ± 8 | 65 ± 36 | 36.28 |
| ***2D6*** | + | + | ***31* ± *6*** | ***69 ± 18*** | ***0.51*** |
| 3A11 | + | + | 58 ± 8 | 91 ± 18 | 21.67 |
| 3B3 | + | + | 252 ± 21 | 99 ± 18 | / |
| ***3B8*** | + | + | ***234 ± 104*** | ***86 ± 12*** | ***0.02*** |
| 3C11 | + | + | 48 ± 10 | 71 ± 11 | 12.69 |
| ***3E6*** | + | + | ***373 ± 295*** | ***243 ± 163*** | ***0.35*** |
| 3E9 | + | + | 248 ± 13 | 143 ± 20 | 1.09 |
| 3F9 | + | + | 98 ± 16 | 141 ± 168 | / |

| | | | | | |
|---|---|---|---|---|---|
| *Antigen binding was evaluated for RBD and trimeric spike protein (Wuhan) via ELISA (in duplicate) and SPR (min. triplicate). Affinity* (*K_{D}; in pM; average ± standard deviation of at least 3 replicates) was determined via SPR. Neutralizing capacity is given in 50% inhibitory concentration* (*IC₅₀*; *in nM) and was measured in triplicate via a SARS-CoV-2 D614G VSV pseudovirus assay. Bold and italic = 8 best neutralizing antibodies;* + = *potent antigen binding (OD ≥ 2) at antibody concentration of 1 µg*/*ml or lower;* / = *no neutralizing activity when tested at a concentration of 5 µg*/*ml or 33.34 nM.* | | | | | |

### Example 2. Evaluation of in vitro efficacy against SARS-CoV-2 variants and other coronavirus species

With the continuous emergence of new SARS-CoV-2 variants, it was crucial to evaluate antigen binding and neutralizing capacity of the selected antibodies against these variants as well. RBD antigens bearing the single mutations E484K, E484Q, N501Y and L452R, present - alone or combined - in multiple SARS-CoV-2 variants (i.e. alpha, beta, gamma, delta and others), as well as RBD antigens bearing the double mutation E484Q, L452R or triple mutation K417N, E484K, N501Y, as present in SARS-CoV-2 variants kappa and beta respectively¹², were used to assess binding and affinity of the 8 most potent antibodies via both ELISA and SPR. In addition, RBD mutations N439K, described as an antibody evasion mutant¹³⁻¹⁵, and RBD Y453F, originating from Danish mink farms and a potential neutralization resistance mutation^{16,17}, were evaluated (an overview of the analyzed RBD single mutations and their presence in SARS-CoV-2 variants is given in Table 3).

**Table 3: overview of single RBD mutants used in this study and their presence in different SARS-CoV-2 variants of concern, variants of interest, variants under monitoring or de-escalated variants. Information retrieved from European Centre for Disease Prevention and Control (https://www.ecdc.europa.eu/en/covid-19/variants-concern)**

| | **SARS-CoV-2 variants** | | | | |
|---|---|---|---|---|---|
| **Mutation** | **Concern** | **Interest** | **Monitored** | **De-escalated^{∗}** | **Other** |
| **N439K** | | | | AV.1 | Ab evasion mutant |
| **L452R** | B.1.617.2 (delta) | C.37 (Lambda) | AY.4.2; | B.1.427/B.1.429 (Epsilon); B.1.617.1 (Kappa); B.1.617.3; A.27; C.16; B.1.526.1 | |
| **Y453F** | | | | | Mink mutant, Ab evasion mutant |
| **E484K** | B.1.351 (beta); P.1 (Gamma) | B.1.621 (Mu) | B.1.1.318; C.1.2 | B.1.525 (Eta); P.3 (Theta); B.1.620; A.28; B.1.526 (Iota); P.2 (Zeta); AT.1; AV.1 | |
| **E484Q** | | | | B.1.617.1 (Kappa); B.1.617.3 | |
| **N501Y** | B.1.351 (beta); P.1 (Gamma) | B.1.621 (Mu) | | B.1.1.7 (Alpha); P.3 (Theta); A.27 | |

| | | | | | |
|---|---|---|---|---|---|
| ^{∗}*These variants of SARS-CoV-2 have been de-escalated based on at least one the following criteria: (1) the variant is no longer circulating, (2) the variant has been circulating for a long time without any impact on the overall epidemiological situation, (3) scientific evidence demonstrates that the variant is not associated with any concerning properties (https:*//*www.ecdc.europa.eu*/*en*/*covid-19*/*variants-concern).* | | | | | |

Interestingly, 7 out of 8 antibodies retained their binding capacity against all analyzed RBD single mutant antigens. One antibody, 2B11, showed reduced binding to RBD L452R and did not bind RBD E484K. This antibody did neither bind the antigens with multiple mutations that included L452R or E484K. Antibody 1C11, although binding with all single mutant antigens, lost its activity against RBD L452R, E484Q double mutant antigen (Table 4 top part). All antibodies that bound the single mutant RBD antigens, still showed high affinities to these antigens, as evident from the equilibrium constants (K_{D}) ranging from 9 ― 647 pM. Only antibodies 1C11 and 2B11 showed an increased K_{D} value for RBD L452R (2940 pM and 3146 pM respectively) (Table 4 bottom part).

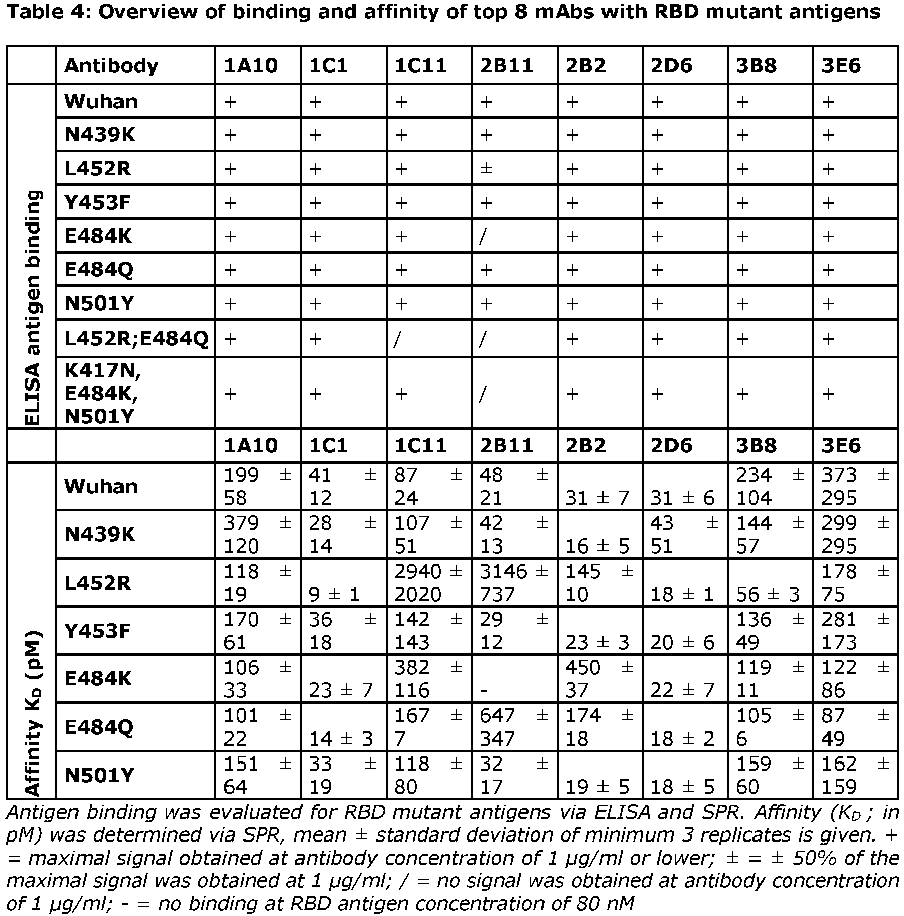

In addition to antigen binding, functional activity against SARS-CoV-2 variants was evaluated in a cytopathic effect neutralization test (CPENT) using live SARS-CoV-2 Wuhan, alpha, beta and gamma virus strains and Vero E6 cells, as well as in a plaque reduction neutralization test (PRNT) using live SARS-CoV-2 delta strain with the same cell line. Infection with SARS-CoV-2 Wuhan and alpha was best neutralized by antibodies 3B8 and 2B2 (IC₅₀ ≤ 0.63 and ≤ 0.16 nM respectively), while SARS-CoV-2 beta infection was best neutralized by antibodies 3B8 and 3E6 (IC₅₀ ≤ 0.32 nM). The same was true for SARS-CoV-2 gamma (IC₅₀ ≤ 0.37 nM for 3B8 and 3E6), although the high standard deviations hinder drawing strong conclusions. SARS-CoV-2 delta was most potently neutralized by 3B8 (IC₅₀ = 0.01 nM), with next in line antibodies 1C1, 2D6, 3E6 (IC₅₀ values between 0.98 ― 1.08 nM). On the other hand, antibodies 2B11 and 1C11 lost their neutralizing activity against SARS-CoV-2 delta (IC50 > 67 nM). Overall, antibody 3B8 was the most potent neutralizing mAb towards all variants, with IC₅₀ values ranging from 0.37 nM to 0.01 nM for neutralization of SARS-CoV-2 gamma and delta strain respectively (Figure 2 and Table 5).

**Table 5: Overview of in vitro neutralizing activity (IC₅₀) towards SARS-CoV-2 Wuhan, alpha, beta, gamma and delta for top 8 mAbs.**

| | **Neutralization** - **IC₅₀ (nM)** | | | | |
|---|---|---|---|---|---|
| **Antibody** | **CPE live virus assay** ― **Wuhan** | **CPE live virus assay** ― **alpha** | **CPE live virus assay** ― **beta** | **CPE live virus assay** ― **gamma** | **PRNT live virus assay** ― **delta** |
| **1A10** | 3.94 | 0.85 | 3.21 | 1.38 | 1.39 |
| **1C1** | 2.31 | 0.55 | 2.56 | 2.53 | 1.05 |
| **1C11** | 1.53 | 0.9 | 5.23 | 3.92 | ND |
| **2B11** | 2.29 | 0.69 | 5.46 | 1.17 | ND |
| **2B2** | 0.63 | 0.16 | 3.02 | 2.31 | 3.97 |
| **2D6** | 3.34 | 0.82 | 2.94 | 1.28 | 0.98 |
| **3B8** | 0.25 | 0.1 | 0.11 | 0.37 | 0.01 |
| **3E6** | 2.19 | 0.85 | 0.32 | 0.19 | 1.08 |

| | | | | | |
|---|---|---|---|---|---|
| *Neutralizing activity (IC₅₀; in nM) was determined via CPE assay for SARS-CoV-2 Wuhan, alpha, beta and gamma and via PRNT assay for SARS-CoV-2 delta.* | | | | | |

The eight selected neutralizing antibodies were also tested for their ability to cross-neutralize other human coronavirus strains using VSV pseudotypes expressing the spike protein from SARS-CoV, Middle East respiratory stress (MERS)-CoV or 229E-CoV on their surface. However, none of the selected antibodies was able to cross-neutralize one of these coronavirus species when used at a concentration of 16.67 nM (or 2.5 µg/ml) (data not shown).

### Example 3. Classification of antibody epitopes

The selected mAbs were classified in different categories based on their epitope specificity using an ELISA cross-competition assay where the antibodies were pairwise tested against one another. Two groups could be distinguished consisting of antibodies 1A10, 1C1 and 2D6 on the one hand and antibodies 2B2, 2B11 and 1C11 on the other hand. Antibodies 3B8 and 3E6 could not be classified in one of these groups (Figure 3). This suggests that the 8 selected mAbs bind to 4 different regions of the RBD antigen.

### Example 4. Potent in vivo efficacy on different SARS-CoV-2 variants in the Syrian golden hamster model

Antibodies, 3B8, 3E6, 2B2 and 1C1, were selected for *in vivo* evaluation based on their *in vitro* neutralizing activity combined with their variability in epitope binding. In a first study, 30 hamsters were divided into 5 groups (6 animals/group) and infected intranasally with 50 µl of SARS-CoV-2 Wuhan virus suspension (containing approximately 2×10⁶ tissue culture infective dose (TCID₅₀)). After 24h, antibodies 3B8, 3E6, 2B2, 1C1 or an IgG1 isotype control were injected intraperitoneally at a dose of 5 mg/kg. All animals were sacrificed at 4 days post infection for analysis of viral RNA and viral infectious titer in the lungs, as well as antibody concentration in serum (Figure 4a). The presence of therapeutic RBD-specific human antibodies circulating in the blood of the treated hamsters was verified through ELISA. This revealed that 5 animals (1 out of 6 for clone 3E6 and 2 out of 6 for clones 2B2 and 1C1) were not successfully injected (Figure 4b). We therefore excluded all animals without detectable serum levels from further analysis. In all groups, antibody treatment reduced viral RNA load in lung tissue as compared to the isotype control group with statistical significance (p < 0.05 for 2B2; p < 0.01 for 3B8, 3E6, 1C1) (Figure 4c). No infectious virus in the lung tissue could be detected in any of the animals treated with antibody 3B8. In animals treated with antibodies 3E6 and 2B2, a low infectious titer was detected in 1 animal, while three out of four animals treated with antibody 1C1 had a low detectable titer. In all groups, the difference was statistically significant (p < 0.01) compared to the isotype control group, thus showing good *in vivo* efficacy for the treatment of SARS-CoV-2 (Wuhan) infection (Figure 4d).

As it was shown previously that SARS-CoV-2 variants of concern beta and delta are more resistant to neutralization by vaccine-induced antibodies or currently available mAb treatments^{6,18,19}, and given that SARS-CoV-2 delta is the most dominant variant worldwide^{20,21}, we decided to evaluate *in vivo* treatment efficacy of our antibodies against both variants as well. A study design similar to the first study was used, with 30 hamsters divided in 5 groups and intranasally infected with 50 µl of SARS-CoV-2 beta²² or SARS-CoV-2 delta suspension (both containing approximately 1×10⁴ TCID₅₀). Animals were injected intraperitoneally with IgG1 isotype control or antibodies 3B8, 3E6, 2B2 and REGN-COV-2 at 5 mg/kg 24h post infection and were sacrificed 4 days post infection, with REGN-COV-2 antibody cocktail being included for benchmarking purposes²³. In the study using SARS-CoV-2 beta, one animal from groups 3B8, 2B2, 1C1 and REGN-CoV-2 as well as two animals from group 3E6 were excluded from analysis because of the absence of detectable antibody serum levels (Figure 5a). In addition, it should be noted that two animals, one from group 3B8 and one from group 2B2, showed very low antibody serum titers, but were not excluded from analysis (indicated by open symbols in the respective groups). Viral RNA load in lung tissue was significantly reduced by treatment with antibody 3B8, 3E6 and REGN-CoV-2 compared to isotype control (p < 0.05 for 3B8 and 3E6; p < 0.01 for REGN-CoV-2), while the reduction observed for 2B2 was not statistically significant (Figure 5b). No infectious viral titer in lung tissue (TCID₅₀) could be detected upon treatment with 3B8, 3E6 or REGN-CoV-2 (p < 0.01 compared to isotype control), while a viral titer was still detectable in four out of five animals treated with 2B2 (Figure 5c). When analyzing the efficacy of our antibodies against SARS-CoV-2 delta infection, a similar picture was observed. Here, only one animal from group 2B2 was excluded because of a lack of detectable serum concentrations (Figure 5d). All treatments resulted in a significant reduction (p < 0.01) of both viral load and infectious viral titers in lung tissue (Figure 5 e and f), with no detectable viral titers in animals treated with antibodies 3B8, 3E6 and REGN-CoV-2. In conclusion, treatment with antibodies 3B8 and 3E6 at 24h post infection resulted in a statistically significant reduction of virus in lung tissue of animals infected with SARS-CoV-2 beta or delta. Although significantly reducing infectious viral titers in lung tissue of animals infected with SARS-CoV-2 delta, antibody 2B2 seems to be less potent as treatment for SARS-CoV-2 beta and delta infection compared to antibodies 3B8 and 3E6.

### Example 5. Dose-dependent therapeutic efficacy of 3B8

Antibodies 3B8 and 3E6 both completely abrogated infectious viral titers after infection with SARS-CoV-2 Wuhan, beta and delta when administered 24h post infection at 5mg/kg. As 3B8 was superior to 3E6 in *in vitro* neutralization experiments, this antibody was used in an *in vivo* dose-response experiment. Hamsters were intranasally infected with SARS-CoV-2 delta (1×10⁴ TCID₅₀). At 24h post infection, isotype control (5 mg/kg) or antibody 3B8 (5 mg/kg; 1 mg/kg, 0.2 mg/kg or 0.04 mg/kg) was administered intraperitoneally. Animals were sacrificed at day 4 post infection. No animals were excluded from analysis based on a lack of detectable antibody serum levels, although one animal from group 0.2 mg/kg had serum levels 3 times lower compared to its group members (animal indicated by open symbol) (Figure 6a). Treatment with a dose of 5 mg/kg and 1 mg/kg of 3B8 significantly reduced the amount of viral RNA detected in lung tissue, and resulted in undetectable levels of infectious virus. When dosing at 0.2 mg/kg, no decrease in viral RNA was seen, but infectious virus was undetectable in lung tissue of 3 out of 6 animals, and was decreased (more than 4 times lower than lower limit of 95% confidence interval from isotype control group) in a fourth animal. In the animal having reduced antibody titers (open symbol), no reduction in viral titer could be observed. Also, at the lowest dose of 0.04 mg/kg, no effect could be observed on viral RNA or infectious viral titer (Figure 6b and c).

### EXAMPLE 6. DNA based vaccination.

3B8 was delivered encoded in plasmid DNA (pDNA) in vivo, as an equimolar mixture of the 3B8 heavy and light chain. Hamsters received an intramuscular pDNA injection in their right & left tibialis anterior and gastrocnemius muscle (pretreated with hyaluronidase), followed by electroporation. This allows for intramuscular production and systemic circulation of 3B8. A series of electrical pulses were thereby delivered at 120V, using a plate electrode. Total pDNA amount delivered per hamster was 600 µg (75 µl pDNA, at 2 µg/µl per muscle). Intramuscular pDNA administration was performed either on day -10, day -7 or day -5 infection (6 animals per timepoint). On day 0, all animals were infected intranasally with SARS-CoV-2 delta (B.1.617.2). Animals of the negative control group were left untreated. Animals were sacrificed at day 4 for analysis of viral RNA levels (left panel) and infectious viral titers (right panel) in their lungs. Injection of hamsters with 3B8 pDNA resulted in a 5-fold log reduction of viral RNA in the lungs, and undetectable infectious viral titers in all animals except for 1, pointing to a successful neutralization of SARS-CoV-2 delta in all groups. In the figure, individual data with median values are shown. Dotted line represents the detection limit. ^{∗∗} = p < 0.01 as determined via Mann-Whitney U test.

### EXAMPLE 7. Materials and methods

### Isolation of PBMCs from convalescent COVID-19 patients

Patients with a PCR-confirmed SARS-CoV-2 infection (>18 years old; recovered from disease) were recruited from the University Hospital Leuven or AZ Groeninge Kortrijk for peripheral blood collection. The study and corresponding experiments were approved by the local ethics committee (S64089) and all patients gave their written informed consent. Immediately after blood sample collection, peripheral blood mononuclear cells (PBMCs) were isolated from EDTA-treated blood by density centrifugation (Lymphoprep, STEMCELL Technologies Inc). After washing, the collected PBMCs were resuspended in freezing medium consisting of 10% (v/v) dimethyl sulfoxide (DMSO; Merck) and 90% fetal bovine serum (FBS; GIBCO) for cryopreservation at -80°C or in liquid nitrogen for long term storage.

### Single-cell sorting of antigen-specific B cells

Human B cells were enriched from cryopreserved PBMCs using the EasySep^{™} Human B Cell Enrichment Kit (STEMCELL Technologies) according to the manufacturer's instructions. After purification, B cells were stained with Zombie Aqua^{™} Fixable Viability Kit (Biolegend) and blocked with FcR Blocking Reagent (Miltenyi Biotec) for 15 minutes on ice. Following the 15 minutes incubation, B cells were washed with FACS buffer (phosphate-buffered saline (PBS) + 2% FBS + 2 mM EDTA) and incubated with biotinylated SARS-CoV-2 RBD protein for 60 minutes at on ice. Histag labeled SARS-CoV-2 RBD (The Native Antigen Company) was biotinylated with the EZ-Link Sulfo-NHS-LC-Biotin kit (Thermofisher Scientific) according to the manufacturer's protocol, corresponding to 1-3 biotin groups per antibody molecule. After the 60 minutes incubation, B cells were washed with FACS buffer and stained with PerCP-cy5.5 anti-human CD19 antibody (Biolegend, 363016), FITC anti-human CD3 antibody (Biolegend, 300306) and PE streptavidin (Biolegend, 405203) for 25 minutes on ice. Subsequently, the stained cells were washed twice with FACS buffer and single-cell sorted by BD Influx^{™} Cell Sorter (BD Biosciences). UltraComp eBeads^{™} Compensation Beads and tosylactivated M-280 Dynabeads (Invitrogen) coupled with SARS-CoV-2 RBD protein according to the manufacturer's instructions were used for compensation. Selection of RBD-specific B cells was performed using the following gating strategy: lymphocytes were selected using FSC and SSC, followed by selection of single cells based on width versus area of FSC and SSC signals. Next, live cells were selected as Zombia Aqua negative cells. Here, B cells were selected as CD19+ CD3- cells and evaluated for RBD surface staining. To allow proper gating of RBD-positive cells, a fluorescence minus one (FMO) control was included (figure 7). Individual B cells were sorted into 96-wells PCR plates (Bioké) containing 2 µL lysis buffer (0.2% (v/v) Triton X-100 + 2U/µL RNaseOUT^{™} Recombinant Ribonuclease (Invitrogen) in UltraPure^{™} DNAse/RNase free distilled water (Invitrogen)) per well. The plates were sealed with a Microseal^{®} 'F' Film (BioRad) and immediately frozen on dry ice before storage at ―80°C for further use.

### Amplification of antibody variable domains

Transcripts of lysed single B cells were denatured and hybridized with a mix of 1 µL 10 mM each nucleotide dNTP-Mix (Invitrogen) and 1 µL 10 µM oligo-dT primerat 72°C for 3 minutes. Subsequently, cDNA synthesis and pre-amplification was performed according to Picelli et al⁴³. cDNA was stored at ―20°C. cDNA was purified with Agencourt AMPure XP beads (Beckman Coulter) according to the manufacturer's protocol and quantified on the BioAnalyzer 2100 instrument (Agilent) and on a Qubit^{™} fluorometer (Thermofisher Scientific). The sequences encoding the immunoglobulin (Ig) heavy- and light-chain variable regions (V_{H} and V_{L}) of IgM and IgG were amplified using reverse primers described by Ozawa et al⁴⁴. and forward primer described by Picelli et al⁴³. The PCR reaction was performed with KAPA HiFi HotStart ReadyMix (Roche) at 95°C for 3 min, followed by 30 cycles at 98°C for 20 seconds, 60°C for 45 seconds and 72°C for 60 seconds and terminated at 72°C for 5 minutes. All PCR products were checked on a 1% agarose gel and purified using the Agencourt AMPure XP beads. Concentrations were measured on a Qubit^{™} fluorometer for Sanger sequencing.

### Recombinant antibody production and purification

### Generation and amplification of expression vectors containing the selected heavy- and light chain sequences

Generation of antibody expression vectors was outsourced (Genscript). The Ig V_{H} and V_{L} sequences were cloned into a pcDNA3.4 expression vector containing human IgG1 constant regions. The plasmid DNA (pDNA) was amplified by transformation in DH5a *E. coli* and subsequent purification using the NucleoBond Xtra Maxi EF kit (Machery - Nagel) according to the manufacturer's instructions. Plasmid purity and integrity was checked on a 1% agarose gel.

### Production of recombinant antibody in HEK93F cells

Recombinant antibodies were transiently transfected and produced in vitro in 293F Freestyle suspension cells (Thermofisher Scientific). Briefly, equal amounts of heavy- and light chain pDNA were mixed with X-tremeGENE HP DNA Transfection Reagent (Roche) in Freestyle^{™} 293 Expression Medium (Thermofisher Scientific) according to the manufacturer's protocol and incubated for at least 15 minutes to allow the pDNA to enter the liposomes. The transfection reagent mixture was added in a dropwise manner to 293F Freestyle suspension cells. The cells were cultured in T175 flasks (Sarstedt, Germany) on an orbital shaker (Thermofischer Scientific) at 135 rpm and 8% CO₂ in a 37°C humidified incubator for 5 days. At day 5, supernatant containing the recombinant antibodies was collected by centrifugation (1,400 × g for 10 minutes at room temperature), filtered through a 0.2 µm filter unit (VWR) and stored at - 20°C.

### Purification of recombinant antibody

Purification of the recombinant antibodies was conducted on a ÄKTAprime plus system (GE Healthcare Life Sciences) at 4°C using a 1 mL HiTrap^{®} Protein A HP prepacked Protein A Sepharose^{®} column (Cytiva). Briefly, the column was first equilibrated in buffer A (20 mM sodium phosphate, 150mM NaCl, pH 7.5) before the sample was loaded on the column. The flow rate for all steps was 1 mL/min. The column was washed with buffer A and buffer B (20mM Sodium Phosphate, 500 mM NaCl, pH 7.5). The recombinant antibodies were eluted from the column with 100 mM sodium acetate, pH 3.5 and the collected fractions were immediately neutralized with 1M Tris, pH 9. The fractions were pooled and dialyzed against sterile-filtered PBS. The ÄKTAprime plus system and all glasswork was treated with 30% hydrogen peroxide (VWR) to reduce the presence of endotoxin.

### Quality control using SDS-PAGE and endotoxin measurements

Purified recombinant antibodies were assessed by SDS-PAGE under nonreducing conditions using the Amersham Phastsystem^{™} (GE Healthcare) according to the manufacturer's instructions. Endotoxin levels were measured on Endosafe^{®}-PTS^{™} (Charles River) according to the manufacturer's instructions.

### ELISA

### Antigen binding

The binding of the purified recombinant antibodies to the following SARS-CoV-2 antigens was assessed via ELISA: spike glycoprotein (S1) RBD-His (REC31849-500, The Native Antigen Company), RBD(N439K)-His (40592-V08H14, Sino Biological), RBD(N501Y)-His (40592-V08H82, Sino Biological), RBD(E484K)-His (40592-V08H84, Sino Biological), RBD(Y453F)-His (40592-V08H80, Sino Biological), RBD(E484Q)-His (40592-V08H81, Sino Biological), RBD(L425R)-His (40592-V08H28, Sino Biological), RBD(L425R,E484Q)-His (40592-V08H88, Sino Biological), RBD(K417N, E484K, N501Y)-His (40592-V08H85, Sino Biological), Spike Glycoprotein (Full-Length)-His (REC31868-500, The Native Antigen Company). Briefly, polystyrene 96-well flat-bottom microtiter plates (Corning costar) were coated with 100 µL/well of 2 µg/mL antigen diluted in PBS and incubated overnight at 4°C. The next day, plates were blocked with 200 µL/well blocking buffer containing 1% (w/v) Bovine Serum Albumin (BSA, Sigma Aldrich) in PBS for 2 hours at room temperature. The plates were then washed six times with wash buffer (0.002% (v/v) Tween-80 (Sigma Aldrich) in PBS). After washing, 100 µL/well of the samples, calibrators and/or controls were added. As a positive and negative control, an anti-SARS-CoV-2 RBD mAb (40150-D004, Sino Biological) and anti-SARS-CoV-2 nucleocapsid antibody (MBS2563841, MyBioSource) were used, respectively. All samples and controls were diluted in PBS + 0.1% (w/v) BSA + 0.002% (v/v) Tween 80 with or without 1,86 g/L EDTA (PTA(E) buffer). After incubation, the plates were washed six times and incubated with 100 µL/well horseradish peroxidase (HRP)-conjugated goat anti-human IgG (Fc-specific, Sigma Aldrich) diluted 1/5000 in PTA buffer. After 1 hour incubation at room temperature, the plate was again washed six times and 100 µL/well substrate (200 µL 40 mg/mL o-Phenylenediamine dihydrochloride 99+% (OPD, Acros Organics BVBA) + 2 µL H₂O₂ (Merck) in 20 mL citrate buffer, pH 5 (0.1 M citric acid monohydrate from Sigma and 0.2 M disodium phosphate dihydrate from Merck)) was added to the plate and incubated for 30 minutes in the dark. The color reaction was stopped with 50 µL/well 4 M H₂SO₄ (Thermofisher Scientific). Optical density (OD) was measured at 492nm with an ELx808 ELISA reader (BioTek). Analysis was performed using Graphpad Prism 9.0 (Graphpad Software).

### Competition assay

Polystyrene 96-well flat-bottom microtiter plates were coated with 4 µg/mL purified recombinant capture antibody in PBS (100 µL/well) and incubated at 4°C. After an overnight incubation, plates were blocked with 200 µL/well blocking buffer for 2 hours at room temperature. The plates were washed six times with washing buffer and 10 ng/mL SARS-CoV-2 RBD protein diluted in PTA buffer (100 µL/well) was added to the plates for a 2-hour incubation at room temperature. After the incubation, the plates were washed and captured antigen was detected with 100 µL/well of 1/100 biotin-conjugated purified recombinant antibodies diluted in PTA buffer. Biotinylation of the purified recombinant mAbs was performed with the EZ-Link Sulfo-NHS-LC-Biotin kit according to the manufacturer's protocol. After a 1-hour incubation at room temperature and washing, biotinylated recombinant antibodies that were able to bind the antigen were detected with 1/10,000 poly-HRP-conjugated streptavidin (Sanquin) diluted in PTA buffer (100 µL/well) and incubated for an additional 30 minutes at room temperature. After a final washing step, 100 µL/well substrate was added to the plate and incubated at room temperature for 30 minutes in the dark at room. The color reaction was stopped with 50 µL/well 4 M H₂SO₄. Optical density (OD) was measured at 492 nm with an ELx808 ELISA reader (BioTek). Very low OD values indicate the detection antibody was not able to bind, suggesting similar epitopes of both coating and detection antibodies, while high OD values indicate both antibodies have a different epitope. Epitope binning graphs were made in Microsoft Excel and clustering was done with ClustVis web tool (https://biit.cs.ut.ee/clustvis/).

### Surface plasmon resonance

Surface Plasmon Resonance (SPR) was used to evaluate the interaction between monoclonal Abs and SARS-CoV-2 antigens (i.e. full-length spike protein, RBD and RBD mutants; see catalog numbers under Methods section "ELISA"). The binding experiments were performed at 25°C on a Biacore T200 instrument (GE Healthcare, Uppsala, Sweden) in HBS-EP+ buffer (10 mM HEPES, 150 mM NaCl, 3 mM EDTA and 0.05% v/v Surfactant P20). First, mouse anti-human IgG (Fc) antibody (Human Antibody Capture Kit, Cytiva) was immobilized on a CM5 chip according to manufacturer instructions. Monoclonal Abs were then captured between 30 and 100 RU. Increasing concentrations of analyte were sequentially injected in one single cycle at a flow rate of 30 µl/min. The dissociation was monitored for 30 min. The chip was finally regenerated with 3M MgCl2 before a new mAb was captured. A reference flow was used as a control for non-specific binding and refractive index changes. Several buffer blanks were used for double referencing. Binding affinities (KD) were derived after fitting the experimental data to the 1:1 binding model in the Biacore T200 Evaluation Software 3.1 using the single cycle kinetic procedure. Each interaction was repeated a least three times.

### Monoclonal antibody neutralization assays

### Production of S-pseudotyped virus and serum neutralization test (SARS2, SARS1, MERS, 229E)

VSV S-pseudotypes were generated as described previously⁴⁵. Briefly, HEK-293T cells (SARS-CoV, SARS-CoV-2 and MERS-S) or BHK-21J cells (229E) were transfected with the respective S protein expression plasmids, and one day later infected (MOI = 2) with GFP-encoding VSVΔG backbone virus (purchased from Kerafast). Two hours later, the medium was replaced by medium containing anti-VSV-G antibody (I1-hybridoma, ATCC CRL-2700) to neutralize residual VSV-G input. After 24 h incubation at 32C, the supernatants were harvested. To quantify nAbs, serial dilutions of serum samples were incubated for 1 hour at 37 °C with an equal volume of S pseudotyped VSV particles and inoculated on Vero E6 cells (SARS-CoV and SARS-CoV-2) or Huh-7 cells (229E and MERS-S) for 18 hours. The percentage of GFP expressing cells was quantified on a Cell Insight CX5/7 High Content Screening platform (Thermo Fischer Scientific) with Thermo Fisher Scientific HCS Studio (v.6.6.0) software. Neutralization IC50 values were determined by normalizing the serum neutralization dilution curve to a virus (100%) and cell control (0%) and fitting in Graphpad Prism (GraphPad Software, Inc.).

### Cell-based cytopathic (CPE) assay and CPE-based virus neutralization test (CPENT) (SARS-CoV-2 Wuhan, alpha, beta, gamma)

The titers of the virus stocks (TCID50) and 50% neutralizing titers (log₁₀CPENT₅₀) were determined using cytopathic effect (CPE)-based cell assays and CPE-based virus neutralization tests (CPENT), respectively, on VeroE6 cells with some modifications. Shortly, 10⁴ VeroE6 cells/well were seeded in 96-well plates one day prior to the titration and inoculated with 10-fold serial dilutions of virus solutions and cultured for 3 days at 37°C. Later, assays first visually scored for CPE and then stained with MTS/Phenazine methosulphate (PMS; Sigma- Aldrich) solution for 1.5 h at 37°C in the dark. Post MTS/PMS staining absorbance was measured at 498 nm for each well. All assays were performed in six replicates and TCID_{50/}ml was determined using Reed and Muench method⁴⁶. CPENT was measured in a similar way as the CPE assays for viral titration with some modifications. In brief, serial dilutions of antibodies were mixed separately with live SARS-CoV-2 Wuhan, alpha, beta and gamma virus strains, incubated at 37°C for 1h, and added to the monolayer of Vero E6 cells. All distinct antibodies were assayed in triplicate in serial dilutions. CPE neutralization was calculated with the following formula: % neutralization activity = % CPE reduction = (OD _{virus + antibody} - OD _{VC}) / (OD _{CC} - OD _{VC}) ^{∗}100 and 50% neutralization titers (CPENT₅₀) were calculated with non-linear regression. Cells either unexposed to the virus or mixed with TCID₅₀ SARS-CoV-2 were used as negative (uninfected) and positive (infected) controls, respectively.

### SARS-CoV-2 plaque reduction neutralization test (PRNT) (SARS-CoV-2 delta)

The PRNT experiments were performed with a SARS-CoV-2 isolate from the B.1.617.2 lineage. This virus was isolated from oropharyngeal swabs taken from a patient in Belgium (EPI_ISL_2425097; 2021-04-20). Virus stocks were prepared by 2x passaging on Vero E6 cells.

Dose-dependent neutralization of distinct antibodies was assessed by mixing the antibodies at different concentrations, with 100 PFU SARS-CoV-2 in DMEM supplemented with 2% FBS and incubating the mixture at 37°C for 1h. Antibody-virus complexes were then added to VeroE6 cell monolayers in 12-well plates and incubated at 37°C for 1h. Subsequently, the inoculum mixture was replaced with 0.8% (w/v) methylcellulose in DMEM supplemented with 2% FBS. After 3 days incubation at 37°C, the overlays were removed, the cells were fixed with 3.7% PFA, and stained with 0.5% crystal violet. Half-maximum neutralization titers (PRNT50) were defined as the antibody concentration that resulted in a plaque reduction of 50%.

### Golden Syrian hamster studies

### SARS-CoV-2

Three SARS-CoV-2 strains were used in this study. BetaCov/Belgium/GHB-03021/2020 (EPI ISL 109 407976|2020-02-03), was recovered from a nasopharyngeal swab taken from an RT-qPCR confirmed asymptomatic patient who returned from Wuhan, China in the beginning of February 2020. A close relation with the prototypic Wuhan-Hu-1 2019-nCoV (GenBank accession 112 number MN908947.3) strain was confirmed by phylogenetic analysis. Infectious virus was isolated by serial passaging on Huh7 and Vero E6 cells⁴⁷; passage 6 virus was used for the study described here. The Beta variant B.1.351 (hCoV-19/Belgium/rega-1920/2021; EPI_ISL_896474, 2021-01-11) was isolated from nasopharyngeal swabs taken from a traveler returning to Belgium in January 2021 who became a patient with respiratory symptoms. The Delta variant, B.1.617.2 (hCoV-19/Belgium/rega-7214/2021; EPI_ISL_2425097; 2021-04-20) was isolated from nasopharyngeal swabs in April 2021 in Belgium through active surveillance. Both strains were subjected to sequencing on a MinION platform (Oxford pore) directly from the nasopharyngeal swabs. Infectious virus was isolated by passaging on VeroE6 cells²²; passage 2 was used for the study described here. Live virus-related work was conducted in the high-containment A3 and BSL3+ facilities of the KU Leuven Rega Institute (3CAPS) under licenses AMV 30112018 SBB 219 2018 0892 and AMV 23102017 SBB 219 20170589 according to institutional guidelines.

### Cells

Vero E6 cells (African green monkey kidney, ATCC CRL-1586) were cultured in minimal essential medium (MEM, Gibco) supplemented with 10% fetal bovine serum (Integro), 1% non-essential amino acids (NEAA, Gibco), 1% L- glutamine (Gibco) and 1% bicarbonate (Gibco). End-point titrations on Vero E6 cells were performed with medium containing 2% fetal bovine serum instead of 10%. Huh7 cells were cultured in Dulbecco's modified Eagle's medium (DMEM, Gibco) supplemented with 10% fetal bovine serum (Integro), 1% bicarbonate (Gibco), 2% HEPES buffer (Gibco). Both cells were kept in a humidified 5% CO₂ incubator at 37°C.

### SARS-CoV-2 infection model in hamsters

The hamster infection model of SARS-CoV-2 has been described before^{47,48}. Female Syrian hamsters *(Mesocricetus auratus)* were purchased from Janvier Laboratories and kept per two in individually ventilated isolator cages (IsoCage N Bio-containment System, Tecniplast) at 21°C, 55% humidity and 12:12 day/night cycles. Housing conditions and experimental procedures were approved by the ethics committee of animal experimentation of KU Leuven (license P065-2020). For infection, female hamsters of 6-8 weeks old were anesthetized with ketamine/xylazine/atropine and inoculated intranasally with 50 µL containing either 2×10⁶ TCID50 SARS-CoV-2 (ancestral Wuhan strain), 1×10⁴TCID50 (Beta B.1.351), or 1×10⁴ TCID50 (Delta B.1.617.2). Antibody treatments were initiated 24 hours post infection by intraperitoneal injection. Hamsters were monitored for appearance, behavior, and weight. At day 4 post-infection, animals were euthanized by intraperitoneal injection of 500 µL Dolethal (200 mg/mL sodium pentobarbital, Vétoquinol SA). Lungs were collected and viral RNA and infectious virus were quantified by RT-qPCR and end-point virus titration, respectively. Blood samples were collected at end-point for pharmacokinetic analysis. No randomization methods were used and confounders were not controlled, though all caretakers and technicians were blinded to group allocation in the animal facility and to sample numbers for analysis (qPCR, titration, and histology).

### SARS-CoV-2 RT-qPCR

Hamster lung tissues were collected after sacrifice and were homogenized using bead disruption (Precellys) in 350 µL TRK lysis buffer (E.Z.N.A.^{®} Total RNA Kit, Omega Bio-tek) and centrifuged (10.000 rpm, 5 min) to pellet the cell debris. RNA was extracted according to the manufacturer's instructions. RT-qPCR was performed on a LightCycler96 platform (Roche) using the iTaq Universal Probes One-Step RT-qPCR kit (BioRad) with N2 primers and probes targeting the nucleocapsid⁴⁸. Standards of SARS-CoV-2 cDNA (IDT) were used to express viral genome copies per mg tissue⁴⁷.

### End-point virus titrations

Lung tissues were homogenized using bead disruption (Precellys) in 350 µL minimal essential medium and centrifuged (10,000 rpm, 5min, 4°C) to pellet the cell debris. To quantify infectious SARS-CoV-2 particles, endpoint titrations were performed on confluent Vero E6 cells in 96- well plates. Viral titers were calculated by the Reed and Muench method⁴⁶ using the Lindenbach calculator and were expressed as 50% tissue culture infectious dose (TCID₅₀) per mg tissue.

### Sample size justification

For antiviral efficacy, we want to detect at least 1 log₁₀ reduction in viral RNA levels in treated subjects compared to the untreated, infected control group. Group size was calculated based on the independent t-test with an effect size of 2.0 and a power of 80% (effect size = delta mean/SD = 1 log₁₀ decrease in viral RNA/0.5 log₁₀), resulting in 5-6 animals/group. Sample sizes maximized considering limits in BSL3 housing capacity, numbers of animals that can be handled under BSL3 conditions, and availability of compounds.

### Ethics

Housing conditions and experimental procedures were done with the approval and under the guidelines of the ethics committee of animal experimentation of KU Leuven (license P065-2020).

### Statistics

All statistical analyses were performed using GraphPad Prism 9 software (GraphPad, San Diego, CA, USA), unless indicated otherwise in the respective methods sections. Statistical significance was determined using the non-parametric Mann Whitney U-test. P-values of <0.05 were considered significant.

### Cited references

1. Ritchie, H. et al. Coronavirus Pandemic (COVID-19). https://ourworldindata.org/coronavirus (2021).
2. Walls, A. C. et al. Cell 181, 281-292.e6 (2020).
3. Wrapp, D. et al. Science (80-. ). 367, 1260-1263 (2020).
4. Taylor, P. C. et al. Nat. Rev. Immunol. 21, 382-393 (2021).
5. Unknown. The Antibody Society: COVID-19 Biologics Tracker. https://www.antibodysociety.org/covid-19-biologics-tracker/ (2021).
6. Planas, D. et al.. Nature 596, 276-280 (2021).
7. Hoffmann, M. et al. Cell 184, 2384-2393.e12 (2021).
8. Corti, D. et al. Cell 184, 3086-3108 (2021).
9. Ryu, D.-K. et al. bioRxiv Prepr. Serv. Biol. (2021).
10. Ryu, D.-K. et al. bioRxiv Prepr. Serv. Biol. (2021).
11. Ryu, D. et al. bioRxiv Prepr. Serv. Biol. (2021).
12. CDC. SARS-CoV-2 Variant Classifications and Definitions. Sep 23 https://www.cdc.gov/coronavirus/2019-ncov/variants/variant-info.html#Interest (2021).
13. Thomson, E. C. et al. Cell 184, 1171-1187.e20 (2021).
14. Chen, J., Gao, K., Wang, R. & Wei, G.-W J. Mol. Biol. 433, (2021).
15. Sanyaolu, A. et al. Ther. Adv. Vaccines 8, 1-10 (2021).
16. Lassaunière, R. et al. Front. Microbiol. 12, 1-9 (2021).
17. Baum, A. et al. Science (80-. ). 369, 1014-1018 (2020).
18. Wang, P. et al. Nature 593, 130-135 (2021).
19. Zhou, D. et al. Cell 184, 2348-2361.e6 (2021).
20. CDC. COVID data tracker: variant proportions. https://covid.cdc.gov/covid-data-tracker/#variant-proportions (2021).
21. ECDC. Variants of interest and concern in the EU/EEA. https://gis.ecdc.europa.eu/portal/apps/opsdashboard/index.html#/25b6e879 c076412aaa9ae7adb78d3241 (2021).
22. Abdelnabi, R. et al. EBioMedicine 68, 103403 (2021).
23. Food and Drug Administration. Fact Sheet for Health Care Providers Emergency Use Authorization Of REGEN-COV. (2021).
24. Connor, B. A. et al.. Int. J. Infect. Dis. 110, 232-234 (2021).
25. Hacisuleyman, E. et al. N. Engl. J. Med. 384, 2212-2218 (2021).
26. Chen, R. E. et al. Nat. Med. 27, 717-726 (2021).
27. Collier, D. A. et al. Nature 593, 136-141 (2021).
28. Kustin, T. et al. medRxiv 1-17 (2021).
29. McCallum, M. et al.. bioRxiv Prepr. Serv. Biol. (2021).
30. Wang, Z. et al. Nature 592, 616-622 (2021).
31. Calcoen, B. et al. [manuscript in preparation*].*
32. Baum, A. et al. Science (80-. ). 370, 1110-1115 (2020).
33. Abdelnabi, R. et al. EBioMedicine 68, 1-26 (2021).
34. Andreano, E. et al. Cell 184, 1821-1835.e16 (2021).
35. Kreye, J. et al. Cell 183, 1058-1069.e19 (2020).
36. Fagre, A. C. et al. Front. Immunol. 11, 1-13 (2020).
37. Piepenbrink, M. S. et al. Cell Reports Med. 2, 100218 (2021).
38. Winkler, E. S. et al. Cell 184, 1804-1820.e16 (2021).
39. Tortorici, M. A. et al. Science (80-. ). 370, 950-957 (2020).
40. Hansen, J. et al. Science (80-. ). 369, 1010-1014 (2020).
41. Jones, B. E. et al. bioRxiv Prepr. Serv. Biol. 1-29 (2020) doi: 10.1101/2020.09.30.318972.
42. Lee, W. S., Wheatley, A. K., Kent, S. J. & DeKosky, B. J. Nat. Microbiol. 5, 1185-1191 (2020).
43. Picelli, S. et al.. Nat. Protoc. 9, 171-181 (2014).
44. Ozawa, T., Kishi, H. & Muraguchi, A. Biotechniques 40, 469-478 (2006).
45. Sanchez-Felipe, L. et al.. Nature 590, 320-325 (2021).
46. Reed, L. & Muench, H.. Am J Epidemiol. 27, 493-497 (1938).
47. Kaptein, S. J. F. et al. Proc. Natl. Acad. Sci. U. S. A. 117, 26955-26965 (2020).
48. Boudewijns, R. et al. Nat. Commun. 11, 1-10 (2020).

## Claims

1. An antibody or antigen binding fragment thereof, specifically binding to the trimeric spike protein of Wuhan Covid-SARS-2 and specifically binding to the RBD domain of all of Wuhan, alpha, beta, gamma and delta variant of Covid-SARS-2 spike protein, **characterized in that:**
said antibody or antigen binding fragment thereof has an IC50 in a SARS-CoV-2 pseudovirus infection lower than 1 nm in a SARS-CoV-2 pseudovirus infection, and **in that**
said antibody or antigen binding fragment thereof has an affinity for RBD domain Wuhan Covid-SARS-2 spike protein with a K_{D} of less than 500 pM, and
**in that**
said antibody or antigen binding fragment thereof has an affinity for trimeric spike protein of Wuhan Covid-SARS-2 with a K_{D} of less than 300 pM.

2. The antibody or antigen binding fragment thereof according to claim 1, wherein
said antibody or antigen binding fragment thereof has an IC50, lower than 0,4, and
said antibody or antigen binding fragment thereof has an affinity for RBD domain Wuhan Covid-SARS-2 spike protein with a K_{D} of less than 400 pM, and said antibody or antigen binding fragment thereof has an affinity for trimeric spike protein of Wuhan Covid-SARS-2 with a K_{D} of less than 300 pM.

3. The antibody or antigen binding fragment thereof according to claim 1 or 2, wherein,
said antibody or antigen binding fragment thereof has an IC50, lower than 0,05 and
said antibody or antigen binding fragment thereof has an affinity for RBD domain Wuhan Covid-SARS-2 spike protein with a K_{D} of less than 250 pM, and said antibody or antigen binding fragment thereof has an affinity for trimeric spike protein of Wuhan Covid-SARS-2 with a K_{D} of less than 100 pM.

4. The antibody or antigen binding fragment thereof according to any one of claims 1 to 3, which is an antibody or antibody fragment comprising:
- a variable heavy chain wherein the sequence of the CDR1, CDR2 and CDR3 region consist respectively of the sequence of GYTFTSHY [SEQ ID NO: 1], IDSSGGGA [SEQ ID NO: 2], and AKAHSTNWYGWFDP [SEQ ID NO: 3] and comprising
a variable light chain wherein the sequence of the CDR1, CDR2 and CDR3 region consist respectively of the sequence SSNIGSNT [SEQ ID NO: 4], NNN and AAWDDGLNGSGWV [SEQ ID NO: 5].

5. The antibody or antigen binding fragment thereof according to claim 4, wherein the variable domain comprises a VH region that has outside the CDR regions at least 95% sequence identity with the amino acid sequence of SEQ ID NO: 22 and a VL region that has outside the CDR regions at least 95% sequence identity to the amino acid sequence of SEQ ID NO: 24.

6. The antibody or antigen binding fragment thereof according to claim 1 or 2, which is an antibody or antibody fragment comprising:
- a variable heavy chain wherein the sequence of the CDR1, CDR2 and CDR3 region consist respectively of the sequence of GVIVSRNY [SEQ ID NO: 6], IYSGGST [SEQ ID NO: 7] and ARWGPNYYGSGSDHYNSYGMDV [SEQ ID NO: 8]
and comprising
a variable light chain wherein the sequence of the CDR1, CDR2 and CDR3 region consist respectively of the sequence QGVSSSS [SEQ ID NO: 9], GAS and QQSGSSPQYT[SEQ ID NO: 10].

7. The antibody or antigen binding fragment thereof according to any one of claims 1 to 7, wherein the antibody is a human antibody.

8. A polynucleotide encoding the antibody or antigen binding fragment thereof in accordance with any one of claims 1 to 7.

9. The antibody or antigen binding fragment thereof according to any one of claims 1 to 7, for use in the treatment and prevention of the beta or delta variant of COVID 19 in an individual.

10. The antibody antigen binding fragment thereof for use in the treatment or prevention of COVID 19 in an individual according to claim 9, wherein the antibody or antibody fragment comprises:
- a variable heavy chain wherein the sequence of the CDR1, CDR2 and CDR3 region consist respectively of the sequence of GYTFTSHY [SEQ ID NO: 1], IDSSGGGA [SEQ ID NO: 2], and AKAHSTNWYGWFDP [SEQ ID NO: 3] and comprising
a variable light chain wherein the sequence of the CDR1, CDR2 and CDR3 region consist respectively of the sequence SSNIGSNT [SEQ ID NO: 4], NNN and AAWDDGLNGSGWV [SEQ ID NO: 5].

11. The antibody antigen binding fragment thereof for use in the treatment or prevention of COVID 19 in an individual according to any one of claims 9 or 10, wherein the variable domain comprises a VH region that has outside the CDR regions at least 95% sequence identity with the amino acid sequence of SEQ ID NO: 22 and a VL region that has outside the CDR regions at least 95% sequence identity to the amino acid sequence of SEQ ID NO: 24.

12. The antibody antigen binding fragment thereof for use in the treatment or prevention of COVID 19 in an individual according to claim 9, comprising a variable heavy chain wherein the sequence of the CDR1, CDR2 and CDR3 region consist respectively of the sequence of GVIVSRNY [SEQ ID NO: 6], IYSGGST [SEQ ID NO: 7] and ARWGPNYYGSGSDHYNSYGMDV [SEQ ID NO: 8]
and comprising
a variable light chain wherein the sequence of the CDR1, CDR2 and CDR3 region consist respectively of the sequence QGVSSSS [SEQ ID NO: 9], GAS and QQSGSSPQYT [SEQ ID NO: 10].

13. A polynucleotide comprising a sequence encoding the antibody or antigen binding fragment thereof according to any one of claims 1 to 7, for use in the treatment and prevention of the beta or delta variant COVID 19.

14. The polynucleotide for use in the treatment or prevention of COVID 19 in an individual according to claim 13, comprising the sequence of SEQ ID NO: 21 and SEQ ID NO: 23.

15. The polynucleotide for use in the treatment or prevention of COVID 19 in an individual according to claim 13 or 14, comprising the sequence of SEQ ID NO: 25 and SEQ ID NO: 27.
